(19) **Europäisches Patentamt** / **European Patent Office** / **Office européen des brevets**

(11) **EP 2 453 985 B1**

(12) **FASCICULE DE BREVET EUROPEEN**

(45) Date de publication et mention
de la délivrance du brevet:
**27.11.2013 Bulletin 2013/48**

(51) Int Cl.:
**A61N 5/10** (2006.01)

(21) Numéro de dépôt: **10732960.9**

(22) Date de dépôt: **13.07.2010**

(86) Numéro de dépôt international:
**PCT/EP2010/060088**

(87) Numéro de publication internationale:
**WO 2011/006907 (20.01.2011 Gazette 2011/03)**

(54) **PROCEDE DE CALCUL DE DOSES DEPOSEES PAR UN RAYONNEMENT IONISANT**

VERFAHREN ZUR BERECHNUNG DER VON DER IONISIERENDENSTRAHLUNG ABGEGEBENEN DOSEN

METHOD FOR CALCULATING DOSES DEPOSITED BY IONIZING RADIATION

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **15.07.2009 FR 0954870**

(43) Date de publication de la demande:
**23.05.2012 Bulletin 2012/21**

(73) Titulaire: **Commissariat à l'Énergie Atomique et aux Énergies Alternatives 75015 Paris (FR)**

(72) Inventeurs:
- **BLANPAIN, Baptiste**
  **F-78000 Versailles (FR)**
- **MERCIER, David**
  **F-91410 Dourdan (FR)**
- **BARTHE, Jean**
  **F-31650 Saint-Orens de Gameville (FR)**

(74) Mandataire: **Lucas, Laurent Jacques**
**Marks & Clerk France**
**Conseils en Propriété Industrielle**
**Immeuble Visium**
**22, Avenue Aristide Briand**
**94117 Arcueil Cedex (FR)**

(56) Documents cités:
**WO-A2-03/092789      US-B1- 6 411 675**

- **BERGMAN ALANAH ET AL: "Direct aperture optimization for IMRT using Monte Carlo generated beamlets" MEDICAL PHYSICS, AIP, MELVILLE, NY, US, vol. 33, no. 10, 14 septembre 2006 (2006-09-14), pages 3666-3679, XP012091877 ISSN: 0094-2405**
- **Blanpain B et al.: "Calcul par réseaux de neurones de la dose déposée en radiothérapie par un faisceau fin dans un volume hétérogène" Majecstic 2007 schedae vol. 2, no. 25, 2007, pages 151-159, XP007911845 caen Extrait de l'Internet: URL:http://hal.archives-ouvertes.fr/docs/0 0/45/29/63/PDF/Article_Majecstic_preprint0 252007.pdf [extrait le 2010-02-18]**

**EP 2 453 985 B1**

**Description**

La présente invention concerne un procédé de calcul de doses déposées par un rayonnement ionisant, par exemple utilisé par un dispositif de traitement thérapeutique par radiothérapie. L'invention peut notamment s'appliquer à une radiothérapie par modulation d'intensité.

La radiothérapie est une technique de traitement du cancer dont un des principes est de détruire une ou des tumeurs. La destruction de la tumeur s'effectue au moyen d'irradiations externes successives par des faisceaux de rayons ionisants, tout en préservant au maximum les tissus sains. Le mode opératoire nécessite une connaissance morphologique de la tumeur ainsi que sa localisation précise dans l'organisme du patient. Ces informations sont obtenues à partir d'images issues d'un scanner ou encore d'images obtenues par résonance magnétique. Un protocole d'irradiation est alors déterminé par un médecin oncologue à l'aide d'un système de planification des traitements. Le protocole d'irradiation définit notamment l'énergie des faisceaux, leur forme, leur position et leur angle d'incidence sur la tumeur. Toute la difficulté consiste à choisir les meilleurs paramètres, c'est-à-dire ceux qui permettront d'atteindre une distribution de dose la plus efficace et la plus sûre pour traiter le patient. La dose représente une quantité d'énergie déposée dans un petit volume de l'organisme du patient. La dose est directement liée à l'impact du traitement en matière de destruction de cellules. La dose est donc la grandeur de référence utilisée en radiothérapie.

En particulier, le principe de la radiothérapie par modulation d'intensité, ou IMRT, est notamment d'effectuer une irradiation selon des directions fixées. La dose totale est déposée en plusieurs dizaines d'irradiations, réalisées sous des angles différents. Pour chaque direction, la forme du faisceau est adaptée à celle de la tumeur. Chaque faisceau peut, de plus, être modulé spatialement en fluence afin notamment de s'adapter aux caractéristiques du patient. La fluence est une quantification du nombre de particules du faisceau, correspondant à l'intensité du faisceau. Il est ainsi possible d'irradier suffisamment la tumeur pour la détruire, tout en limitant l'irradiation des parties saines pour minimiser les effets secondaires indésirables de la radiothérapie. Ainsi, les médecins oncologues indiquent pour chaque zone à traiter une dose minimale pour la tumeur ou une dose maximale pour les organes sains. Les médecins oncologues proposent donc à partir de leur expérience un protocole d'irradiation. Pour valider le protocole d'irradiation, les médecins peuvent disposer d'outils de calcul d'une répartition de la dose dans le patient selon le protocole préconisé.

Plusieurs méthodes existantes permettent de calculer la dose déposée par des rayonnements ionisants dans un patient. Ces méthodes peuvent être regroupées en deux catégories :

- des méthodes très précises, telles que la méthode de Monte Carlo, demandant des temps de calcul trop longs pour être utilisées en clinique et notamment lors de l'élaboration d'un protocole de traitement. En effet, un des objectifs d'un oncologue est qu'à la fin d'une consultation avec un patient, il puisse immédiatement fixer des dates de séances de radiothérapie. L'oncologue dispose donc d'environ une vingtaine de minutes pour valider le protocole qu'il a mis au point. De plus, si une optimisation du protocole est souhaitée par l'oncologue, il est nécessaire que les calculs de doses ne prennent que quelques secondes, les calculs de doses pouvant ainsi être itérés dans le cadre du processus d'optimisation. De telles méthodes précises sont trop coûteuses en temps de calcul pour être utilisées dans ce cadre.

- des méthodes plus rapides telles que les méthodes Clarkson, Pencil Beam, Noyaux, sont en général très insuffisantes en termes de précision. Une mauvaise précision des résultats obtenus peut mettre la vie du patient en danger. En effet, un patient est composé de différents matériaux : chair, eau, os, de composition électronique et de densité différente. Les transitions entre deux matériaux traversés successivement par un faisceau peuvent conduire à un déséquilibre électronique au niveau de l'interface entre les deux matériaux qui modifie significativement la dose déposée aux abords des interfaces. Les méthodes rapides ont une gestion du déséquilibre électronique aux interfaces approximative, voire inexistante. Par exemple, aux abords d'interfaces complexes, les erreurs de calcul peuvent dépasser 15% de la dose réellement déposée. En cas de sous-dosage des rayonnements sur la tumeur, cela peut impliquer la survie de certaines cellules et donc l'inefficacité du traitement. En cas de surdosage sur des organes critiques, comme la moelle épinière ou le nerf optique, cela peut impliquer des dommages irréversibles sur ces organes. De plus, même si ces méthodes sont plus rapides que des méthodes de type Monte-Carlo, elles n'offrent pas un temps de calcul suffisamment court pour être utilisables pour une optimisation automatique des planifications de traitement.

La méthode dite de Monte-Carlo est notamment décrite par Andreo P. en 1991, dans le document : « Monte Carlo techniques in medical radiation physic, Phys. Med. Biol. 36, 861-920 », et par Salvat F., Fernandez-Varea J., Acosta E. & Sempau J. en 2006, dans le document : « PENELOPE-2006, A Code System for Monte Carlo Simulation of Electron and Photon Transport, in NEA 6222, ISBN: 92-64-02301-1 ». La méthode de Monte-Carlo exploite un modèle physique microscopique et statistique des interactions rayonnement-matière : des trajectoires de particules d'un faisceau sont simulées par des tirages aléatoires successifs de probabilités d'interactions entre les particules et le matériau qu'elles pénètrent. Afin que le résultat soit statistiquement acceptable, un grand nombre de trajectoires sont simulées :

de l'ordre de $10^7$. La dose libérée par les différentes interactions est alors cumulée dans les voxels du volume bombardé par les particules. Un voxel peut être défini comme le plus petit élément d'un espace tridimensionnel numérisé : c'est en quelque sorte un pixel volumétrique. Le grand nombre de calculs de trajectoires nécessite un temps de calcul très important, de l'ordre de plusieurs heures sur un ordinateur classique.

Une méthode dite « Phase Space Evolution » est notamment décrite par Huizenga, H. & Storchi, P. R. M. en 1989, dans « Numerical calculation of energy deposition by broad high-energy electron beams, Phys. Med. Biol. 34, 1371-96 », et par Janssen, J. J.; Riedeman, D.; Morawska-Kaczynska, M.; Storchi, P. R. M. & Huizenga, H. en 1994, dans « Numerical calculation of energy deposition by high-energy electron beams: III. Three-dimensional heterogeneous media, Phys. Med. Biol. 39, 1351-66 ». Cette méthode consiste, dans un volume donné, à transporter de voxel en voxel des flux d'électrons, échantillonnés selon leur énergie et leur angle de déplacement. Ceci nécessite de disposer, dans des bases de données, pour chaque groupe d'électrons d'énergie donnée, arrivant sur un voxel avec une direction donnée, de modèles donnant une distribution en énergie et en angle des électrons ressortant du voxel. Les modèles donnant la distribution en énergie sont notamment appelés fonctions de distribution. Les fonctions de distribution sont pré-calculées à partir d'équations physiques, mais elles peuvent également l'être à partir de simulations de type Monte-Carlo telles que décrites précédemment. La méthode « Phase Space Evolution » utilisant des données pré-calculées, elle est plus rapide qu'une méthode de Monte-Carlo. Cependant le temps de calcul qu'elle nécessite reste encore très important.

Une autre approche, assez similaire sur le principe physique, consiste à résoudre les équations macroscopiques de transport des électrons et photons par la technique des éléments finis. Par exemple Gifford K.A., Horton J.L., Wareing T.A., Failla G and Mourtada F présentent cette approche avec l'équation linéaire de transport de Boltzmann dans 'Comparison of a finite-element multigroup discrete-ordinates code with Monte Carlo for radiotherapy calculations', Physics in Medicine and Biology, 51, 2253-2265, 2006. La grandeur physique d'intérêt est la fluence qui est ici une fonction multivariable définie en tout point, pour toute direction, pour les différents types de particules considérées et pour tout niveau énergétique de chaque type de particule. Cette fonction est dans la pratique et selon le principe même de la technique des éléments finis, quantifiée selon les directions et les niveaux d'énergie des particules. Ladite fonction est spatialement projetée sur une base locale de décomposition comme des polynômes de Legendre. La résolution est globale. La dose dans un petit volume est ensuite calculée à partir de l'expression de la fluence. La quantification selon les directions et les niveaux d'énergie ne pouvant pas être trop grossière sous peine d'être trop imprécise, cette méthode requiert une taille mémoire conséquente et les temps de calcul sont encore importants.

Afin d'accélérer les calculs de dose, il a été proposé d'utiliser des distributions en trois dimensions, de doses pré-calculées par une méthode Monte-Carlo sur des fantômes homogènes afin de reconstruire la dose sur des fantômes hétérogènes. Des fantômes sont des représentations numériques du corps humain, utilisés pour simuler les effets des rayonnements sur l'organisme.

Parmi les méthodes de calcul plus rapides, des méthodes de calcul considèrent implicitement qu'il y a un équilibre électronique sur l'ensemble du fantôme. L'équilibre électronique est atteint en un volume infinitésimal d'un matériau, lorsque qu'il y a autant d'électrons entrants dans le volume et y déposant leur énergie que d'électrons qui en sortent. C'est par exemple le cas pour des méthodes décrites par Wong J. & Purdy J. en 1990) dans 'On methods of inhomogeneity corrections for photon transport', Med. Phys. 17, 807-14, dans le document AAPM de 2004 : Tissue inhomogeneity corrections for megavoltage photon beams, AAPM Report No 85 (College Park, MD: AAPM). Ces méthodes proposent une mise en correspondance entre un point d'un profil de dose hétérogène et un point situé à la même profondeur radiologique dans un profil de dose homogène, c'est-à-dire un point ou la fluence des deux faisceaux est la même. Cependant, cette méthode est bien trop imprécise aux interfaces entre des matériaux différents, puisqu'elle ne gère pas les différences de flux électroniques aux interfaces.

Une autre méthode, dite de Clarkson, est notamment décrite par Clarkson, J. en 1941 dans 'A note on depth doses in fields of irregular shape', Brit. J. Radiol. 14, 265-8 et par Cunningham J. R. en 1972 dans, 'Scatter-air ratios', Phys. Med. Biol. 17, 42-51. Dans la méthode de Clarkson, les calculs de dose sont effectués à deux niveaux, celui de la dose primaire et celui de la dose secondaire. Une dose primaire peut être définie comme une dose déposée par une première interaction par exemple de photons composant le faisceau de particules avec la matière, puis par les électrons issues de la première interaction. Une dose secondaire peut être définie comme une dose déposée par des photons secondaires via leur interaction avec la matière ou l'interaction avec la matière des électrons qu'ils ont produits. Un photon est dit secondaire s'il ne compose pas le faisceau initial mais a été produit suite à une interaction d'un autre photon ou d'un électron avec la matière, eux-mêmes pouvant composer le faisceau initial ou résulter d'une cascade d'interactions initiée par une première interaction d'un photon du faisceau initial avec la matière. A partir d'un découpage de la section d'un faisceau large en secteurs triangulaires, cette méthode reconstruit la dose en additionnant les contributions de chaque secteur. On peut ensuite adapter la méthode de Clarkson au cas d'un profil de faisceau ayant une fluence hétérogène. Une fluence hétérogène peut être obtenue par un filtre en coin par exemple. La méthode de Clarkson consiste également dans ce cas en un découpage en secteurs angulaires du faisceau. Enfin, les fantômes hétérogènes peuvent être également traités en prenant en compte les matériaux que traversent le faisceau, découpé en secteurs et en sous-secteurs, puis ceux que traversent les particules secondaires. Ces différentes adaptations ajoutent une forte complexité aux calculs tout en ne garantissant pas une bonne précision.

Des méthodes dites par convolutions et/ou superposition sont notamment décrites par : Mackie T. R., Scrimger J. W. & Battista J. J. en 1985 dans 'A convolution method of calculating dose for 15-MV x rays', Med. Phys. 12, 188-96 ; Ahnesjô A. en 1989 dans 'Collapsed cone convolution of radiant energy for photon dose calculation in heterogeneous media', Med. Phys. 16, 577-92 ; Tillikainen L., Helminen H., Torsti T. ,Siljamäki S., Alakuijala J., Pyyry J. & Ulmer, W. en 2008 dans 'A 3D pencil-beam-based superposition algorithm for photon dose calculation in heterogeneous media', Phys. Med. Biol. 53, 3821-39. Une approche classique, dite du « point kernel », signifiant « point noyau », utilise des noyaux de dépôt d'énergie. Les noyaux de dépôt d'énergie donnent une répartition de la dose secondaire autour d'un point d'interaction de photons primaires. Les noyaux sont pré-calculés via des simulations de Monte-Carlo. Ainsi la dose en trois dimensions peut être obtenue par convolution d'un tel noyau avec une densité d'interaction des photons primaires. La densité d'interaction des photons primaires peut être obtenue par un tracé du faisceau en profondeur. Cependant, cette convolution nécessitant un temps de calcul important, différentes accélérations ont été proposées. L'une d'elle est la méthode dite « collapsed cone convolution », signifiant littéralement convolution du cône d'effondrement, décrite par Ahnesjô. La méthode « collapsed cone convolution » consiste à modéliser des noyaux sous forme de fonctions exponentielles. Une telle modélisation rend possible une procédure accélérée du calcul de convolution. Cependant, malgré l'accélération des calculs, ceux-ci durent de l'ordre d'une minute, ce qui n'est pas adapté aux conditions d'utilisation souhaitées. Une autre approche classique pour accélérer la convolution de la méthode « point kernel » est nommée « pencil beam », signifiant littéralement « faisceau crayon ». La méthode « pencil beam », décrite par Tillikainen, consiste à pré-intégrer les noyaux le long d'un axe vertical pour former la dose déposée par un faisceau de section infinitésimale. La reconstruction de la dose pour un faisceau complet consiste alors à utiliser une convolution du noyau « pencil beam » avec un profil de fluences d'entrée du faisceau complet. Des corrections apportées à cette méthode permettent d'obtenir une précision adéquate du calcul de doses au niveau des interfaces entre deux matériaux différents. Toutefois, les temps de calcul restent encore de l'ordre de la minute.

Différents auteurs ont proposé d'utiliser des réseaux de neurones artificiels pour apprendre des profils de dose dans des matériaux homogènes afin de les restituer pour des matériaux hétérogènes. Parmi les méthodes utilisant des réseaux de neurones, une méthode est décrite par Vasseur A., Makovicka L., Martin E., Sauget M., Contassot-Vivier S. & Bahi J. en 2008 dans 'Dose calculations using artificial neural networks: A feasibility study for photon beams', Nucl. Instr. and Meth. in Phys. Res. B 266, 1085-93. Etant donné qu'il n'est pas envisageable, en raison de la complexité du problème, d'utiliser les capacités de généralisation des réseaux de neurones pour qu'ils restituent directement la dose en milieu hétérogène, Vasseur a proposé de coupler des réseaux de neurones avec des techniques d'ajustement de la dose aux interfaces. Un milieu hétérogène est un milieu composé de plusieurs matériaux différents. Ainsi des modèles semi-physiques ont été constitués. Cependant, du fait des hypothèses de continuité électronique aux interfaces entre matériaux différents, les méthodes décrites par Vasseur ne peuvent modéliser correctement les faisceaux étroits de l'IMRT. Une amélioration de cette méthode est proposée par B. Blanpain, D. Mercier, J. Barthe., dans le document « Calcul par réseaux de neurones de la dose déposée en radiothérapie par un faisceau fin dans un volume hétérogène », Actes de la manifestation des jeunes chercheurs en sciences et technologies de l'information et de la communication MaJeSTIC 2007, Caen, 29-31 octobre 2007. L'amélioration proposée est une extension des méthodes utilisant des réseaux de neurones, à des faisceaux étroits, pour lesquels il n'y a pas d'équilibre électronique latéral, ce qui entraine d'importantes discontinuités au niveau des interfaces entre des matériaux différents. Cependant, la précision et la rapidité de telles méthodes ne sont pas suffisantes pour l'application visée.

Un but de l'invention est notamment de calculer très rapidement la dose déposée dans patient par un protocole d'irradiation, tout en ayant une précision suffisante pour ne pas mettre en danger le patient. A cet effet, l'invention a pour objet un procédé de calcul de doses déposées par au moins un faisceau de particules ionisantes sur des voxels d'un fantôme d'un patient. Le fantôme peut être maillé. Chaque maille du fantôme peut comporter des voxels d'un même matériau. Le procédé de calcul peut comporter au moins les étapes suivantes pour chaque faisceau :

- une première étape de calcul d'au moins une fonction analytique de répartition de doses déposées par le premier faisceau par exemple pour chaque maille d'un ensemble de mailles du fantôme ;
- une deuxième étape de calcul de doses sur plusieurs voxels du maillage, la valeur de la dose pour un voxel étant notamment la valeur de la fonction analytique de répartition de doses de la maille à laquelle appartient le voxel, à la position du voxel dans la maille.

La première étape peut comporter :

- un premier calcul de fonctions analytiques pour des premières mailles du fantôme traversées par le premier faisceau, les fonctions analytiques ainsi obtenues peuvent être des modèles piliers ;
- un deuxième calcul de fonctions analytiques pour des deuxièmes mailles du fantôme, non traversées par le premier faisceau, par exemple par diffusion des modèles piliers, en parcourant notamment de proche en proche les deuxièmes mailles du fantôme, en partant des mailles traversées par le premier faisceau, pour obtenir des modèles de diffusion par exemple pour les mailles de l'ensemble de mailles qui ne sont pas traversées par le premier faisceau.

L'ensemble des mailles du fantôme comporte des mailles pour chacune desquelles par exemple au moins une des valeurs de la fonction analytique sur la maille, est supérieure à un seuil donné.

Une fonction analytique peut être composée d'au moins deux fonctions :

- une première fonction de projection associant par exemple une première position p d'une maille à une deuxième position p' dans un fantôme par exemple d'un matériau homogène, ledit matériau homogène ayant des caractéristiques semblables aux caractéristiques du matériau des voxels de la maille ;
- une deuxième fonction modèle associant par exemple à la deuxième position p' dans le fantôme du matériau homogène une dose y étant déposée par un deuxième faisceau semblable au premier faisceau. Un modèle de diffusion peut être composé de trois fonctions :
- la première fonction de projection ;
- la deuxième fonction modèle ;
- une troisième fonction de validité associant par exemple à une troisième position dans une des deuxièmes mailles, un degré de pondération appliqué à la deuxième fonction modèle.

Un calcul de fonctions analytiques de répartition de doses peut être effectué pour deux mailles adjacentes de différents matériaux, la deuxième interface entre les deux mailles pouvant être traversée de manière oblique par le premier faisceau, en utilisant par exemple une décomposition du premier faisceau en plusieurs sous-faisceaux. Le calcul de fonctions analytiques pouvant être effectué pour chaque sous-faisceau de la même façon que pour un faisceau. Il est également possible de ne pas utiliser de décomposition du faisceau ou d'appliquer des projections déformantes différentes assurant une continuité de la fluence aux interfaces.

Un calcul de fonctions analytiques de répartition de doses déposées par le premier faisceau peut être effectué pour deux mailles adjacentes de différents matériaux, le premier faisceau se propageant par exemple de manière sensiblement parallèle à la première interface. Le calcul de fonctions analytiques peut comporter un calcul d'une fonction analytique par sous-faisceau. Le premier faisceau peut être décomposé en plusieurs sous-faisceaux. Le calcul de fonctions analytiques peut être effectué pour chaque sous-faisceau de la même façon que pour un faisceau.

Une fonction analytique de répartition de doses déposées par le premier faisceau peut être obtenue par une somme pondérée des fonctions analytiques associées à chaque sous-faisceau du premier faisceau, ladite pondération dépendant d'une première position p d'une maille.

La pondération peut être déduite d'une normalisation de premiers coefficients issus d'une fonction de forme Gaussienne.

La pondération peut être déduite d'une normalisation de deuxièmes coefficients issus d'une fonction de la forme d'une fonction de Bell.

Des éléments correctifs peuvent être appliqués à une fonction analytique de répartition de doses pour une cinquième maille, de matériau différent par rapport à une sixième maille adjacente à la cinquième maille, lesdits éléments correctifs pouvant modéliser une discontinuité électronique à proximité d'une troisième interface entre la cinquième maille et la sixième maille adjacente.

Les éléments correctifs peuvent être basés sur des modèles « shutdown », signifiant littéralement modèles d'arrêt.

Des éléments correctifs pour une maille sont basés sur une somme pondérée des fonctions analytiques de la maille et des fonctions analytiques des mailles adjacentes à la maille, ladite pondération dépendant d'une première position p dans la maille.

La dose déposée par un faisceau dans le fantôme de matériau homogène est donnée par un modèle de base pré-calculé en utilisant une distribution de dose obtenue par une simulation selon une méthode de Monte-Carlo.

L'invention a notamment pour principal avantage d'obtenir un temps de calcul de doses très réduit pour un ensemble de points d'un fantôme.

D'autres caractéristiques et avantages de l'invention apparaîtront à l'aide de la description qui suit, donnée à titre illustratif et non limitatif, et faite en regard des dessins annexés qui représentent :

- la figure 1 : un ordinogramme des grandes étapes du procédé selon l'invention ;
- la figure 2a : un premier exemple d'un calcul de doses sur des points du fantôme ;
- la figure 2b : un deuxième exemple d'un calcul de doses sur des points du fantôme ;
- la figure 3a : plusieurs étapes possibles d'un calcul des fonctions analytiques pour les mailles d'un fantôme ;
- la figure 3b : plusieurs étapes possibles d'un calcul d'une fonction analytique pour une maille et un faisceau donnés ;

- la figure 4 : un exemple d'une méthode de projection d'un modèle en milieu homogène sur un milieu hétérogène ;
- la figure 5 : un faisceau traversant une maille de matériau homogène ;
- la figure 6 : un faisceau positionné sur une interface entre deux mailles comportant deux matériaux différents ;
- la figure 7 : un faisceau traversant deux mailles comportant deux matériaux différents, de manière sensiblement orthogonale à l'interface entre les deux matériaux ;
- la figure 8 : un faisceau traversant des mailles de façon oblique.

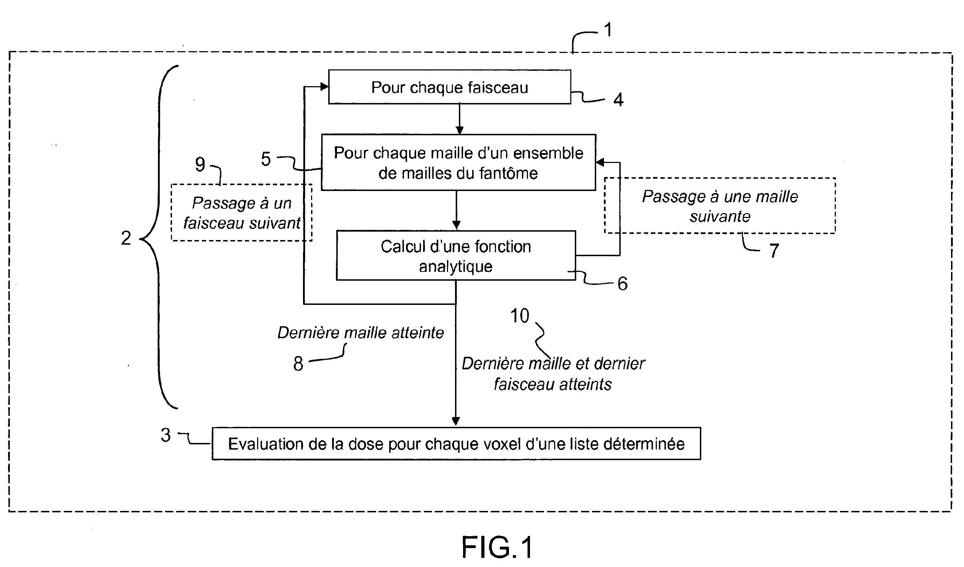

La figure 1 représente plusieurs étapes générales du procédé de calcul de doses de rayonnements ionisants, déposées par au moins un faisceau de particules. Un tel faisceau de particules peut être utilisé par un dispositif de traitement thérapeutique d'un cancer. Le procédé de calcul selon l'invention peut notamment être mis en oeuvre par un système de planification de traitement par radiothérapie. Un tel système peut notamment être utilisé par un médecin oncologue au cours d'une consultation afin d'établir un protocole de traitement thérapeutique utilisant la radiothérapie.

Le principe d'un traitement par des rayonnements ionisants est de détruire une tumeur en utilisant un ou plusieurs faisceaux de particules de manière simultanée comme des photons, des électrons, des hadrons.

Dans le cadre du mode de réalisation présenté à titre d'exemple, les faisceaux de particules sont des faisceaux étroits de photons de section constante et avec une fluence homogène. Toutefois, la présente invention ne se limite pas à de tels faisceaux, l'invention peut être appliquée à des faisceaux hétérogènes et divergents.

Les photons ont pour principal rôle de mettre en mouvement des électrons, lesdits électrons sont responsables de la majeure partie des ionisations et du dépôt de dose. C'est pourquoi les photons sont dits indirectement ionisants, et les électrons directement ionisants.

Une grandeur physique utilisée pour caractériser un faisceau de photons est la fluence. La fluence est définie par le nombre de photons traversant une unité de surface. La fluence $\Phi_0$ d'un faisceau de photons pénétrant dans un matériau homogène est atténuée selon une loi exponentielle. A une profondeur $z$ dans le matériau pénétré par le faisceau, la fluence en photons n'ayant pas encore interagi est donnée par la relation suivante :

$$\Phi_z = \Phi_0 e^{-\mu z} \qquad\qquad (1000)$$

Le coefficient $\mu$ est appelé coefficient d'atténuation linéique. Il est proportionnel à la densité électronique du matériau, c'est-à-dire au nombre d'électrons par unité de volume, et varie également avec l'énergie du photon.

Les atomes ionisés libèrent des électrons qui causent d'autres ionisations sur leur parcours, avant de s'arrêter lorsqu'ils ont perdu toute leur énergie. Contrairement aux photons, les électrons interagissent continûment avec la matière, perdant ainsi très rapidement leur énergie. Leur parcours est de longueur bornée, alors qu'un photon a une probabilité non nulle de traverser n'importe quelle épaisseur de matière.

Une grandeur d'intérêt utilisée pour quantifier des dépôts d'énergie par des rayonnements ionisants est la dose D. La dose D est une grandeur locale égale à l'énergie déposée, rapportée à la masse m. Cette énergie déposée est la différence entre l'énergie qui entre dans un petit volume de masse $dm$, et celle qui en ressort : $dE$.

$$\text{D'où } D = dE / dm \qquad\qquad (1001)$$

Le procédé de calcul selon l'invention utilise notamment un fantôme d'un patient, c'est à dire une représentation matricielle en trois dimensions d'une partie du corps du patient. Dans un fantôme, chaque voxel est caractérisé par un matériau et/ou une densité électronique. Le fantôme du patient est ensuite maillé. Une maille correspond à un regroupement de voxels adjacents. Tous les voxels d'une même maille sont notamment relatifs à un même matériau et à une même densité électronique. Le maillage peut être régulier ou irrégulier, les mailles pouvant être de dimensions et de formes variables. Dans la suite de la description et à titre d'exemple, le maillage utilisé comporte des mailles de forme parallélépipède-rectangle.

Le procédé selon l'invention utilise également des modèles de base de dépôt de dose dans des matériaux homogènes. Un modèle base de dépôt de dose peut exister pour chaque milieu homogène, c'est à dire pour chaque matériau composant le corps humain. Par ailleurs, un modèle d'un premier milieu non disponible peut être déduit d'un modèle d'un deuxième milieu, proche en densité électronique du premier milieu. Le modèle relatif au deuxième milieu peut être obtenu par une opération de compression ou de dilatation du modèle du premier milieu. L'opération de compression ou de dilatation est appelée une mise à l'échelle ou « scaling ». Un modèle de base peut être obtenu à partir d'une distribution de dose obtenue de manière connue par une méthode de type Monte Carlo par exemple, par un paramétrage d'un outil de régression. L'étape de paramétrage est parfois appelée aussi étape d'apprentissage. Un tel outil de régression peut utiliser un réseau de neurones, une fonction spline, un régresseur à vecteurs support communément nommé SVR pour support vector régression, ou un interpolateur dans un tableau de valeurs. Les modèles de bases sont notamment définis pour les même conditions : un faisceau avec toujours une même section, une même fluence unitaire, une même distribution spectrale en énergie, arrivant depuis le vide, de manière sensiblement orthogonale, sur un demi-espace contenant le matériau considéré. Il est également possible de définir des modèles de base pour des faisceaux de sections différentes, de distribution spectrales différentes, ou d'autres variations de conditions expérimentales, en produisant autant de modèles par matériaux qu'il existe de cas à prendre en compte.

Sur la figure 1 sont représentées deux grandes étapes 2, 3 du procédé de calcul de doses 1 selon l'invention.

Une première grande étape 2 du procédé de calcul de doses 1 peut être une étape de calcul d'une formulation analytique d'une répartition de dose pour chacune des mailles d'un ensemble de mailles du fantôme et pour chacun des faisceaux. Le faisceau global peut être décomposé en plusieurs faisceaux ou sous-faisceaux, de fluences différentes par exemple, plusieurs faisceaux peuvent irradier en même temps la tumeur. L'ensemble de mailles est propre à chaque faisceau ou sous-faisceau. L'ensemble de mailles correspond aux mailles irradiées par le faisceau ou sous-faisceau. La formulation analytique peut être une fonction ou un modèle analytique de répartition de dose permettant de calculer directement la dose déposée notamment par un faisceau donné en tout point d'une maille donnée. Par la suite, pour la formulation analytique de répartition de dose, on emploie indifféremment les termes fonction analytique ou modèle analytique. Chaque maille de l'ensemble de mailles peut donc être associée à un ou plusieurs modèles analytiques selon le nombre de faisceaux ou sous-faisceaux interagissant avec le matériau de la maille. Un modèle, ou fonction, analytique correspondant à un faisceau prend en paramètre une position d'un point dans la maille et fournit en sortie la dose déposée par le faisceau en ce point de la maille. La première grande étape 2 peut donc, par exemple, comporter les traitements suivants : pour chaque faisceau 4, et pour chaque maille de l'ensemble de mailles 5, une fonction analytique est calculée 6. Une fois la détermination d'une fonction analytique pour une maille effectuée, on passe à une maille suivante 7 dans l'ensemble de mailles propre au faisceau et on calcule une fonction analytique relative à la maille suivante. Une fois toutes les mailles de l'ensemble de mailles parcourues, c'est à dire que la dernière maille de l'ensemble de mailles est atteinte 8, l'algorithme passe à un faisceau ou sous-faisceau suivant 9. Une fois tous les faisceaux parcourus, c'est à dire la dernière maille de l'ensemble des mailles du dernier faisceau traitée 10, on passe à la deuxième grande étape 3 d'évaluation de la dose pour chaque voxel d'une liste de voxels prédéterminée. La liste de voxels peut, par exemple, contenir l'ensemble des voxels du fantôme ou seulement certains voxels présentant un intérêt particulier du point de vue médical par exemple. Si aucun modèle analytique n'est associé à une maille, la dose déposée en chaque voxel de cette maille est considérée comme nulle.

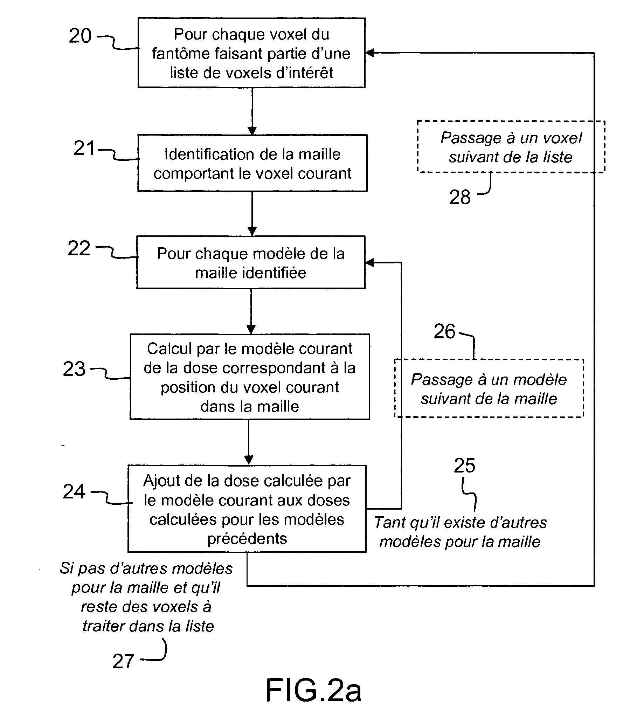

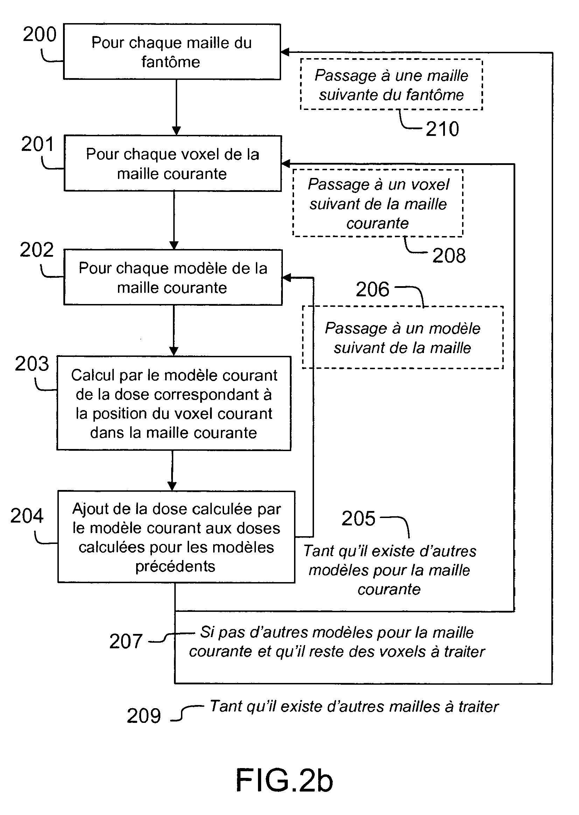

Les figures 2a et 2b représentent des exemples d'étapes possibles pour une évaluation de la dose 3 en des points du fantôme, telle que représentée sur la figure 1. Selon le principe de superposition de doses, une dose déposée en une position particulière du fantôme est la somme des doses déposées par chaque faisceau ou sous-faisceau. Chaque faisceau ou sous-faisceau correspond à un modèle analytique pour chaque maille du fantôme.

La figure 2a représente des étapes possibles de calcul de la dose en différents points du fantôme, chaque point étant représenté par un voxel. La figure 2a représente particulièrement un calcul de la dose pour une liste de voxels d'intérêt, prédéfinie. Pour chaque voxel de la liste 20 de voxels d'intérêt, il s'agit d'identifier la maille du fantôme 21 à laquelle appartient un voxel en cours de traitement dans la liste : le voxel courant. Ensuite, pour chaque modèle analytique associé à la maille identifiée 22, la valeur de la dose est calculée pour le voxel courant 23. La valeur de la dose calculée est ensuite ajoutée aux valeurs des doses calculées par les modèles analytiques précédemment utilisés pour le voxel courant 24. Tant qu'il existe des modèles analytiques non utilisés pour calculer la dose au voxel courant de la maille identifiée 25, le calcul de la dose est réitéré par le passage à un modèle suivant 26 pour la maille identifiée et par un calcul de la dose par le modèle suivant, succédant au modèle courant 23. Ensuite la nouvelle dose obtenue par le modèle suivant est ajoutée 24 aux doses précédemment obtenues pour le voxel courant. Ensuite, lorsque la dose a été calculée pour chaque modèle analytique de la maille identifiée pour le voxel courant 27, le même calcul est opéré pour un voxel suivant le voxel courant 28 dans la liste des voxels d'intérêts 20, et ceci jusqu'à ce que la dose ait été calculée pour tous les voxels de la liste d'intérêt.

La figure 2b représente d'autres étapes possibles de calcul de la dose en différents points du fantôme. Les étapes représentées sur la figure 2b peuvent être mises en oeuvre lorsque la dose est calculée en tout point du fantôme ou de façon régulière comme par exemple tous les n points, n étant un nombre entier supérieur à un par exemple. Les étapes représentées sur la figure 2b ont l'avantage, dans ce cas, de rendre le calcul de la dose plus rapide du fait qu'il n'est pas utile d'identifier la maille comportant le voxel courant. Pour chaque maille du fantôme 200, pour chaque voxel d'une maille en cours de traitement du fantôme 201, ou pour chaque nième voxel de la maille courante, pour chaque modèle associé à la maille courante 202, le calcul de la dose correspondant à la position du voxel courante dans la maille courante est effectué par le modèle courant 203. Ensuite la dose calculée par le modèle courant est ajoutée à la dose calculée par les modèles précédents pour le voxel courant, dans la maille courante 204. Tant qu'il existe des modèles associés à la maille courante 205 par lesquels la dose au voxel courant n'a pas été calculée, un modèle suivant de la maille devient le modèle courant 206 et le calcul de la dose 203 est effectué puis ajouté aux doses précédemment calculées pour le voxel courant de la maille courante 204. Et ainsi de suite jusqu'à ce que tous les modèles de la maille courante aient calculé leur contribution à la dose du voxel courant. Ensuite, si il reste des voxels à traiter 207, un voxel suivant devient le voxel courant 208 et les modèles de la maille courante calculent la dose à la position du voxel courant comme décrit précédemment. Ensuite, lorsque la dose a été calculée pour tous les voxels ou tout les n voxels de la maille courante et qu'il reste des mailles du fantômes pour lesquelles les doses n'ont pas été calculées 209, les doses sont calculées pour les voxels de la maille suivante, qui devient alors la maille courante 210. Ainsi les doses sont calculées pour chaque voxel du fantôme, ou pour chaque nième voxel du fantôme.

Avantageusement, le calcul de la dose sur chaque voxel est réduit à l'estimation d'une formule analytique rendant ainsi ce calcul très rapide. De plus comme l'algorithme permet de calculer la dose uniquement en des points pour lesquels c'est nécessaire, le temps de calcul dépend du nombre de voxels sélectionnés. Avantageusement plus le nombre de voxel est faible, plus le calcul est rapide. Ceci permet donc d'adapter le temps de calcul à des contextes d'utilisation du procédé selon l'invention selon que l'on dispose de plus ou moins de temps pour obtenir un résultat exploitable.

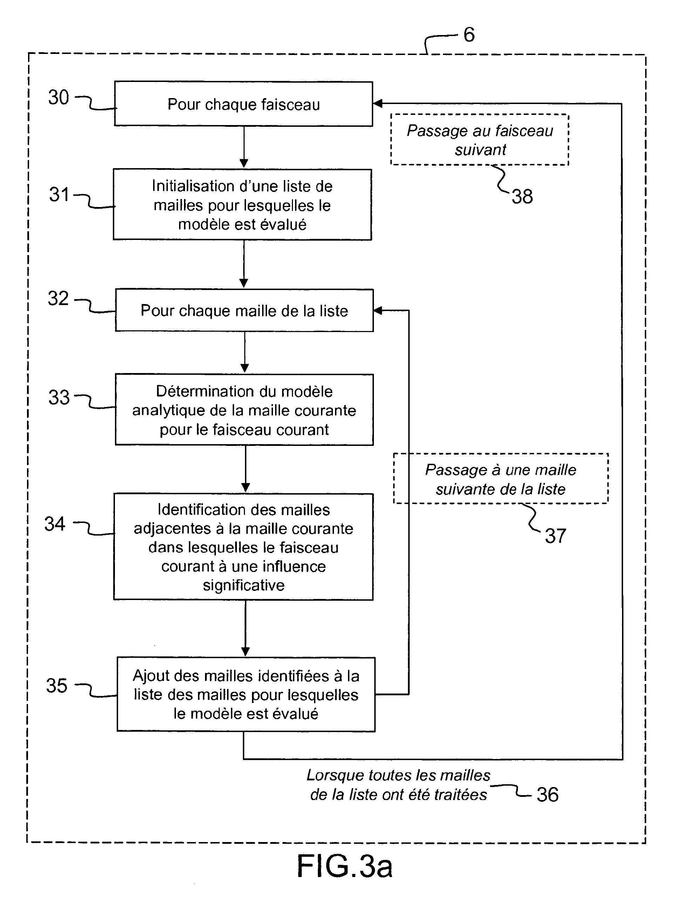

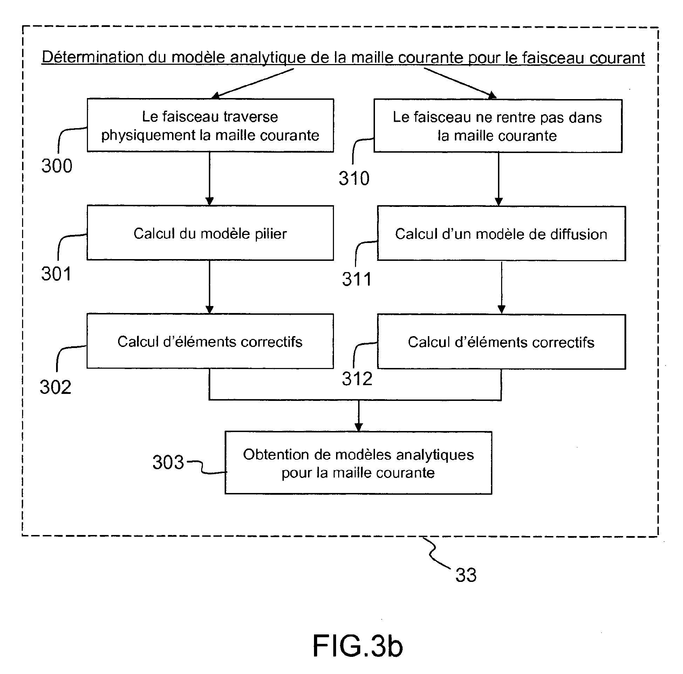

Les figures 3a et 3b représentent des étapes de la détermination d'une formulation analytique pour chaque maille et chaque faisceau 6 telle que représentée sur la figure 1. La détermination d'une formulation analytique pour chaque maille et chaque faisceau est un processus itératif représenté sur la figure 3a. Ainsi pour chaque faisceau 30, une liste de mailles, pour lesquelles le modèle est évalué 31, est initialisée. Les mailles de la liste initiale sont les mailles par lesquelles le faisceau courant entre dans le fantôme. Aux mailles de la liste sont associées des informations sur chaque faisceau qui les traversent comme l'orientation du faisceau, la fluence du faisceau, ainsi que des informations de propagation neutre. Les informations de propagation neutre traduisent le fait que le faisceau n'a traversé aucun matériau jusqu'à sa pénétration dans la maille. Les informations de propagation neutre traduisent également le fait que la dose sur la surface extérieure de la maille est nulle puisque l'on peut supposer qu'à l'extérieur du fantôme, le faisceau traverse le vide. Tant que la liste des mailles pour lesquelles le modèle est calculé n'est pas vide, pour chaque maille de la liste 32, le modèle analytique de la maille courante pour le faisceau courant est déterminé 33. La détermination du modèle analytique de la maille courante pour le faisceau courant 33 se base notamment :

- sur des caractéristiques du faisceau courant, auquel le modèle est associé ;
- sur des caractéristiques relatives à la propagation du faisceau courant dans la maille courante.

Ensuite, les mailles adjacentes à la maille courante, dans lesquelles le faisceau courant à une influence significative 34, sont identifiées et ajoutées à la liste des mailles pour lesquelles un modèle analytique est évalué 35. L'influence significative du faisceau courant dans une maille courante peut être caractérisé par une valeur seuil donnée pour au moins une des valeurs de la fonction analytique sur la maille courante. L'identification des mailles adjacentes à la maille courante, dans lesquelles les effets du faisceau doivent être propagés, s'effectue dans le sens de propagation du faisceau et en fonction de la valeur de la dose calculée à l'interface entre la maille courante et chaque maille adjacente à la maille courante. Si la valeur de la dose sur une interface entre la maille courante et une maille adjacente peut être considérée comme négligeable en tout points de l'interface, la dose est alors considérée comme négligeable dans la maille adjacente en question. Les mailles pour lesquelles la dose est considérée comme négligeable ne sont donc pas ajoutées à la liste de mailles. Les mailles ajoutées à la liste des mailles sont associées à des paramètres du faisceau ainsi qu'à des paramètres de propagation du faisceau. Chaque maille de la liste de mailles pour lesquelles le modèle est évalué sont traitées tour à tour 37 jusqu'à ce que la liste de mailles soit vide. Lorsque toutes les mailles de la liste ont été traitées 36, on traite le faisceau ou sous-faisceau suivant si il existe. Le sous-faisceau suivant devient le sous-faisceau courant 38 et le traitement recommence par l'initialisation d'une liste de mailles pour lesquels le modèle doit être évalué 31, pour le faisceau courant.

La figure 3b représente différentes étapes de la détermination 33 du modèle analytique de la maille courante pour le faisceau courant. Pour déterminer le modèle analytique relatif à une maille pour un faisceau donné, deux cas peuvent être distingués :

- un premier cas 300 pour lequel le faisceau courant, ou une partie du faisceau courant, traverse physiquement la maille courante ;
- un deuxième cas 310 pour lequel le faisceau courant ne traverse pas la maille courante.

Dans le premier cas où le faisceau courant, ou une partie du faisceau courant, traverse physiquement la maille, le modèle analytique peut être déterminé en deux temps.

Dans un premier temps, un modèle pilier est calculé 301. Un modèle pilier représente la dose déposée dans la maille courante en supposant que les mailles adjacentes à la maille courante ont la même composition que la maille courante elle-même. Le modèle pilier est notamment le modèle qui, dans une maille a, en général, le plus d'influence sur le calcul de la dose en tout point de la maille. Le modèle pilier peut être défini à partir d'un modèle de dépôt de dose dans un matériau homogène. Le modèle de dépôt de dose dans un matériau homogène est aussi appelé modèle en milieu homogène ou modèle de base. Un modèle en milieu homogène correspond à des conditions expérimentales précédemment décrites. Le faisceau arrive sur le matériau de manière sensiblement orthogonale au matériau, depuis le vide, sur un demi-espace contenant le matériau considéré. De plus le faisceau est de fluence unitaire.

Le modèle pilier est obtenu par une projection du modèle en milieu homogène. La projection s'effectue par une formule qui associe à une première position donnée p de la maille courante une deuxième position p' dans un matériau homogène tel que : la dose déposée à chaque première position p dans la maille courante, sous l'hypothèse que les mailles adjacentes ont même composition que la maille courante, est égale au produit de la fluence du faisceau par la dose déposée en milieu homogène à la deuxième position p' associée.

Si une partie seulement du faisceau traverse la maille courante, un facteur correctif, lié à la proportion du signal traversant la maille et à des considérations géométriques, est appliqué au modèle pilier.

Dans un deuxième temps, des premiers éléments correctifs 302 du modèle pilier peuvent être calculés. Les premiers éléments correctifs permettent de tenir compte de déséquilibres électroniques provoqués par un éventuel changement de matériau aux interfaces entre les mailles adjacentes. Les premiers éléments correctifs prennent en compte le modèle pilier de la maille courante mais également le modèle pilier d'une maille adjacente à la maille courante, située en amont de la maille courante par rapport au sens de propagation du faisceau, et éventuellement le modèle pilier d'une maille  adjacente à la maille courante située en aval de la maille courante par rapport au sens de propagation du faisceau. La prise en compte du modèle pilier d'une maille située en aval est utile pour tenir compte d'un phénomène de rétrodiffusion du faisceau. L'élément correctif est décrit plus en détail par la suite. L'élément correctif peut prendre différentes formes comme un modèle additif ou des pondérations appliquées aux différents modèles piliers.

Dans le deuxième cas, le faisceau ne rentre pas dans la maille courante 310. La dose déposée ne provient alors que du phénomène de diffusion des électrons et des photons secondaires dans le matériau. Dans ce cas, le modèle analytique de la maille est un modèle résultant d'une opération de diffusion 311 des modèles piliers définis pour les mailles proches de la maille courante. Le modèle ainsi obtenu peut être nommé modèle de diffusion. Des modèles piliers de mailles différentes n'influencent pas le modèle analytique de la maille courante dans les mêmes zones. La diffusion d'un modèle pilier d'une maille adjacente s'effectue en définissant une zone de validité du modèle de diffusion dans la maille courante ou en définissant des poids affectés aux modèles de diffusion, lesdits poids dépendant de la position dans la maille et valant zéro là où le modèle de diffusion n'est pas valide. Dans ce cas, il n'existe pas de modèle pilier sur l'ensemble de la maille courante. Des deuxièmes éléments correctifs peuvent ensuite être déterminés 312. Les deuxièmes éléments correctifs ont les mêmes origines physiques et sont similaires dans leurs formes aux premiers éléments correctifs 302.

Avantageusement, si le fantôme est segmenté en mailles de tailles importantes, l'étape de calcul du modèle analytique pour chaque maille 33, représenté sur la figure 3a, est très rapide. En particulier le temps de calcul des modèles piliers 301, des modèles de diffusion 311, ou des premiers et deuxièmes éléments correctifs 302, 312 sont identiques pour deux fantômes ayant un même maillage mais dont l'un comporte deux fois plus de voxels dans chaque direction que l'autre fantôme.

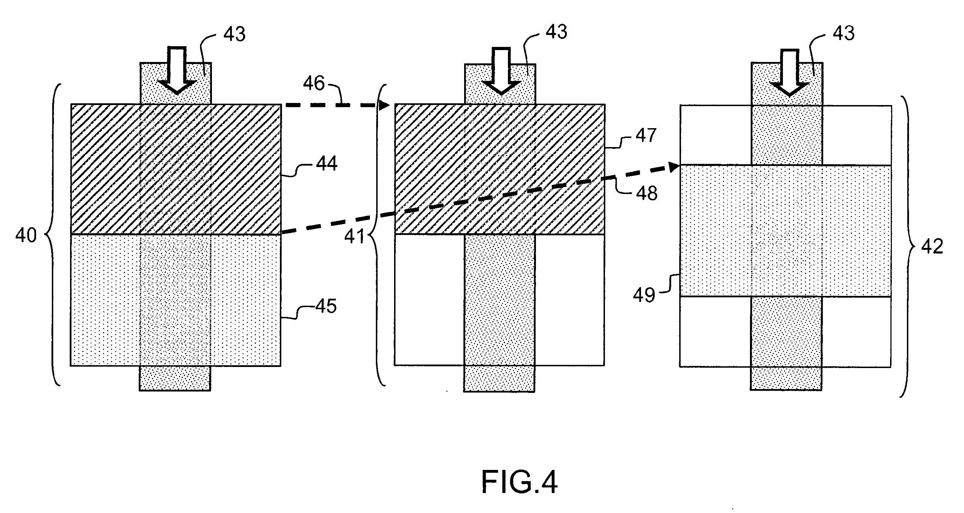

La figure 4 illustre le principe de la projection vers un modèle homogène pour obtenir des modèles analytiques pour chacune des deux mailles 44, 45, d'un fantôme hétérogène 40. Le fantôme hétérogène 40 comporte deux matériaux différents. Une première maille 44 du fantôme  hétérogène comporte un premier matériau homogène, par exemple de l'eau. Une deuxième maille 45 comporte un deuxième matériau homogène, par exemple de l'os. La figure 4 représente un faisceau 43 pénétrant dans le fantôme hétérogène 40 par la première maille 44. La figure 4 représente également le même faisceau, 43 pénétrant un premier fantôme homogène comportant un troisième matériau homogène 41 composé d'eau, c'est-à-dire composé du même matériau que la première maille 44. La figure 4 représente aussi le faisceau 43 pénétrant un deuxième fantôme homogène comportant un quatrième matériau homogène 42 composé d'os, c'est-à-dire composé du même matériau que la deuxième maille 45. Les modèles piliers utilisés pour les mailles 44, 45 peuvent être des compositions de deux fonctions :

- une première fonction de projection qui associe à la première position p en trois dimensions dans une maille 44, 45, la deuxième position p' dans un fantôme homogène 41, 42 dont le matériau possède les mêmes caractéristiques que le matériau de la maille 44, 45 ;
- une deuxième fonction modèle qui associe à la deuxième position p' dans le fantôme homogène 41, 42, la dose qui y est déposée par un faisceau 43 identique au faisceau 43 entrant dans la maille 44, 45 mais de fluence unitaire.

Donc pour chaque matériau de chaque maille 44, 45, une projection 46, 48 de l'espace des positions de la maille 44, 45 vers un espace 47, 49 de positions d'un fantôme homogène de matériau correspondant, soumis au même faisceau 43, est définie. Par exemple, une première projection 46 part d'un premier espace de positions de la première maille 44 vers un deuxième espace de positions 47 dans un premier fantôme de matériau homogène 41. Le matériau de la première maille 44 est du même type que le matériau du premier fantôme homogène 41. Une deuxième projection 48 part d'un troisième espace de positions de la deuxième maille 45 vers un quatrième espace de positions 49 dans un deuxième fantôme de matériau homogène 42. Le matériau de la deuxième maille 45 est du même type que le matériau du deuxième fantôme homogène 42. La dose déposée dans un fantôme hétérogène 40 est donc calculée à partir d'une position équivalente ou correspondante dans un fantôme homogène 41, 42 irradié par le même faisceau 43.

Les deux fonctions peuvent être obtenues de la manière suivante :

- la première fonction de projection dépend des paramètres du faisceau 43, comme la position, l'orientation, les paramètres de propagation du faisceau ;
- le modèle de doses déposées dans le fantôme homogène 41, 42 de même matériau que la maille 44, 45 est pré-paramétré, de façon à être disponible immédiatement pendant le procédé de calcul de doses selon l'invention : à cette fin des distributions de doses en trois dimensions pour le faisceau d'intérêt 43, dans différents matériaux composant le fantôme 40 du patient, sont pré-calculées puis utilisées pour l'apprentissage des modèles de doses avant de mettre en oeuvre le procédé selon l'invention.

Les distributions de doses pré-calculées peuvent être générées par exemple par une méthode de Monte Carlo. Les outils de régression utilisés pour le modèle de dose peuvent être par exemple des réseaux de neurones, des fonctions splines, des régresseurs à vecteurs supports, des interpolateurs entre les valeurs d'un tableau. Les distributions de doses pré-calculées peuvent être générées d'autres façons par exemple en effectuant une interpolation de valeurs relevant de données réelles. D'autres modes de génération peuvent également être envisagés, un critère important étant la qualité des données permettant de s'appuyer sur des distributions de doses réalistes.

Avantageusement, la plus grande partie des calculs liés à des équations physiques de transport de particules et d'interaction entre le rayonnement et la matière est intégrée dans les modèles de base.

Les projections 46, 48 peuvent être des fonctions linéaires transformant des coordonnées en trois dimensions dans un premier espace tridimensionnel, en coordonnées en trois dimensions dans un deuxième espace tridimensionnel.

Soit $f$ une fonction de projection pour une maille. Soit $m$. une position du premier espace en trois dimensions, liée à la maille. La position $m$ peut être représentée par un vecteur en colonne à quatre éléments. Le quatrième élément vaut conventionnellement un, et permet de définir un ensemble de similitudes, c'est-à-dire des rotations, des homothéties, des translations ou toute composition de ces transformations, par une matrice.

$$m = \begin{pmatrix} x \\ y \\ z \\ 1 \end{pmatrix} \qquad (1002)$$

Soit $r$ la position dans le repère du fantôme homogène, $r$ est représentée par un vecteur en colonne à trois éléments. Le repère du fantôme homogène peut être orienté de façon à ce que la troisième composante du vecteur $r$ soit orientée selon un axe dans le sens de propagation du faisceau dans le fantôme homogène. La troisième composante du vecteur $r$ correspond alors à une profondeur de pénétration du faisceau dans le fantôme homogène.

$$r = \begin{pmatrix} x' \\ y' \\ z' \end{pmatrix} \qquad (1003)$$

La fonction linéaire de projection $f$ peut être définie par un produit matriciel. La matrice de projection $P$, donnant la projection, peut s'écrire de la façon suivante :

$$P = \begin{pmatrix} a & b & c & \alpha \\ d & e & f & \beta \\ g & h & i & \gamma \end{pmatrix} \qquad (1004)$$

Les lettres latines *a, b, c, d, e, f, g, h, i* représentent des coefficients donnant une orientation des deux repères l'un par rapport à l'autre. Les lettres grecques $\alpha, \beta, \gamma$ donnent les positions des repères l'un par rapport à l'autre, c'est donc l'expression d'un décalage d'un repère par rapport à l'autre.

La fonction de projection *f* peut alors s'écrire :

$$r = f(m) = Pm \qquad\qquad (1005)$$

L'utilisation de la fonction de projection *f* pour établir des modèles analytiques relatifs aux mailles est expliquée dans la description de la figure suivante.

Une alternative à la composition d'une première fonction de projection et d'une deuxième fonction modèle pour déterminer des fonctions analytiques peut être d'approximer directement la dose par une combinaison d'une fonction exponentielle pour le profil en profondeur et d'une fonction en forme de cloche pour le profil latéral. Le paramètre de la fonction exponentielle dépend uniquement du matériau traversé et les paramètres de la fonction cloche dépendent du matériau traversé et de la taille du faisceau. Il n'y a alors plus de projection puisque de simples calculs de distance suffisent : distance entre la première position p et le plan orthogonal au faisceau, passant par le point d'entrée dans la maille courante de l'axe du faisceau courant pour la fonction exponentielle et distance entre la première position p et l'axe du faisceau pour la fonction cloche. D'autres fonctions analytiques peuvent également être envisagées.

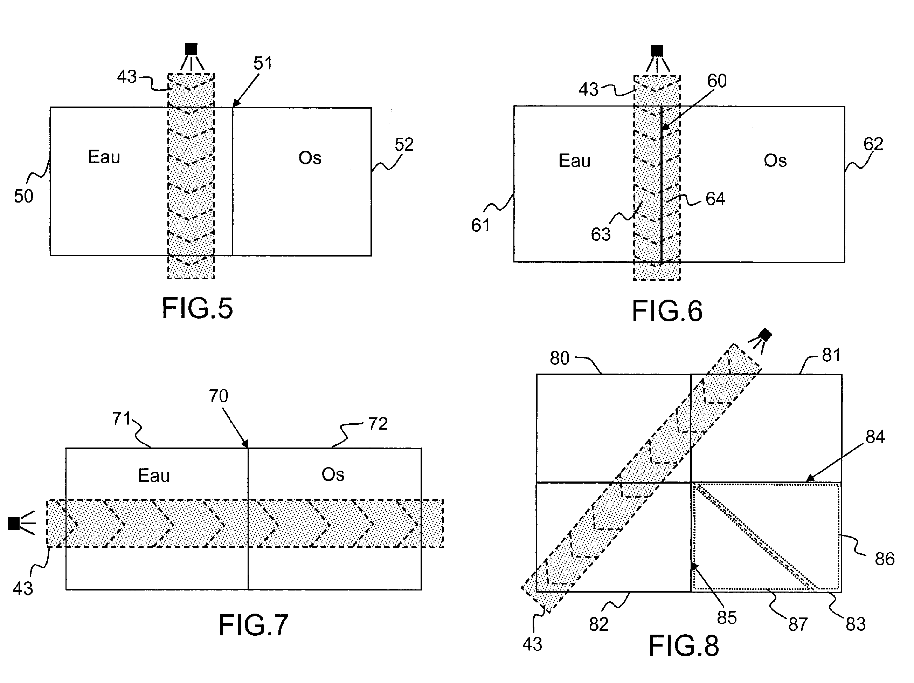

La figure 5 représente le faisceau 43 traversant une troisième maille 50 de façon sensiblement parallèle à une première interface 51 entre la troisième maille 50 et une quatrième maille 52. L'ensemble du faisceau 43 traverse la troisième maille 50. Une étape préalable à la détermination des modèles analytiques pour les mailles du fantôme du patient et pour le faisceau courant 43 peut être un calcul des matrices de projection pour chaque maille traversée par un faisceau en fonction des caractéristiques du faisceau sur chaque maille. Par exemple dans le cas représenté sur la figure 5, des caractéristiques du faisceau 43 peuvent être : une position du faisceau 43, des dimensions et une profondeur radiologique déjà parcourue par le faisceau 43. La profondeur radiologique représente une épaisseur d'eau qu'il faudrait faire traverser à un faisceau initial, identique au faisceau 43, pour que le faisceau 43, à l'entrée de la maille, ait la même fluence que le faisceau initial, après que le faisceau initial ait traversé l'épaisseur d'eau. Le faisceau initial est le faisceau 43 pris en sortie de la tête d'irradiations d'une source d'irradiation. Le principe de détermination d'un modèle pilier comme décrit précédemment est le suivant : à une position donnée dans une maille donnée est associée une position dans une distribution de dose déposée par le même faisceau dans un fantôme homogène du même matériau que celui de la maille donnée. Le même modèle homogène et la même formule de projection sont utilisés pour toute la maille. Le principe de détermination d'un modèle pilier tel que décrit précédemment n'est applicable que si le faisceau ou une partie du faisceau traverse la maille considérée, ce qui est le cas pour la troisième maille 50.

Soient *m* et *r* les positions respectivement dans la maille et dans le fantôme homogène. Les coefficients *a, b, c, d, e, f, g, h, i* et les décalages $\alpha, \beta, \gamma$ de la matrice de projection *P* sont déterminés en fonction de caractéristiques géométriques du faisceau et de caractéristiques de propagation du faisceau.

Les coefficients *a, b, c, d, e, f, g, h, i* sont directement déterminés à partir d'une expression du trièdre directeur du faisceau 43 dans le référentiel de *m*, c'est-à-dire le référentiel de la maille. Le référentiel de la maille peut être identique au référentiel du fantôme du patient. Le trièdre directeur du faisceau est le triplet de vecteurs unitaires qui définissent les axes du faisceau qui sont équivalents aux directions canoniques pour le modèle homogène. (*a, b, c*) peut donc être un premier vecteur directeur normé, exprimé dans le référentiel de la maille qui correspond à la direction de la première composante de *r*. (*d, e, f*) peut être un deuxième vecteur directeur normé exprimé dans le référentiel de la maille qui correspond à la direction de la deuxième composante de *r*. (*g, h, i*) peut être un troisième vecteur directeur normé exprimé dans le référentiel de la maille, correspondant à la direction de la troisième composante de *r* qui est selon la convention prise ici la profondeur donc l'axe de propagation du faisceau. Les décalages $\alpha, \beta$ et $\gamma$ sont alors calculés à partir d'un point particulier pour lequel on connaît simultanément *m* et *r*. Par exemple, lorsque cela est possible, on peut prendre un point se trouvant exactement au centre du faisceau et sur une surface d'entrée du faisceau dans la maille. En effet, le point étant au coeur du faisceau, sa projection est telle que les deux premières composantes de *r* sont nulles, ce qui détermine complètement $\alpha$ et $\beta$. De plus, il est possible de calculer la profondeur de ce point dans le modèle homogène à partir de la profondeur qu'il a dans le modèle précédent. La profondeur est la même si les matériaux des deux mailles sont similaires et sinon elle est déduite par un produit avec le ratio des densités électroniques des deux matériaux. La profondeur calculée permet alors d'identifier $\gamma$. La matrice de projection *P* est ainsi complètement déterminée.

La figure 5 permet également d'illustrer le calcul d'un modèle de diffusion pour des mailles dans lesquelles une dose est déposée mais qui ne sont pas traversées par un faisceau, telles que la quatrième maille 52. Les modèles de diffusion ne sont calculés que pour les mailles dont la dose déposée par le faisceau courant n'est pas considérée comme négligeable 34 et qui par conséquent ont été ajoutées à la liste de mailles 35 tel que représenté sur la figure 3a. La première interface 51 sépare deux matériaux différents, par exemple l'eau dans la troisième maille 50 et l'os dans la quatrième maille 52.

Le faisceau 43 passe dans la troisième maille 50 composée d'eau. On cherche à calculer la dose dans la quatrième maille 52 composée d'os. La dose déposée dans la quatrième maille 52 résulte principalement de particules mises en mouvement dans la troisième maille 50, se diffusant dans la quatrième maille 52.

Un modèle de diffusion peut être une composition de trois fonctions :

- une troisième fonction de projection qui associe à une troisième position q en trois dimensions dans la quatrième maille 52, une quatrième q' dans un fantôme homogène ;
- une quatrième fonction modèle qui associe à la deuxième position q' dans le fantôme homogène, la dose qui y est déposée par un faisceau 43 identique au faisceau 43 entrant dans la troisième maille 50 mais de fluence unitaire ;
- une cinquième fonction de validité qui associe à la troisième position q un degré de validité pondérant la quatrième fonction modèle.

Une autre approche peut être de ne pas utiliser de fonction de validité et de travailler par combinaison des différentes fonctions de diffusions arrivant dans la maille depuis un faisceau unique, en utilisant par exemple une fonction maximum, une fonction moyenne.

Au moins trois calculs différents peuvent être mis en oeuvre afin de déterminer le modèle de diffusion de la quatrième maille 52 qui n'est pas traversée par le faisceau 43.

Un premier calcul peut utiliser un troisième fantôme homogène traversé par le faisceau 43, de même matériau que le matériau de la troisième maille 50. La distribution de dose dans le troisième fantôme homogène est utilisée comme quatrième fonction modèle du modèle de diffusion de la quatrième maille 52. La distribution de doses dans le troisième fantôme homogène est utilisée pour calculer le modèle analytique donnant la contribution à la dose déposée dans la quatrième maille 52 par les effets du faisceau dans la troisième maille 50. La fonction de projection du modèle de diffusion de la quatrième maille 52 vers le troisième fantôme homogène peut être initialisée comme pour un modèle pilier pour les coefficients $a, b, c, d, e, f, g, h, i$ de la matrice de projection $P$. Une première mise à l'échelle est alors effectuée afin de prendre en compte les différences de propagation entre les matériaux du troisième fantôme homogène et le matériau de la quatrième maille 52. La première mise à l'échelle est latérale : elle s'applique aux deux premières dimensions de $r$ et donc aux coefficients $a, b, c, d, e, f$ de la matrice de projection $P$. La première mise à l'échelle peut être réalisée par exemple selon le rapport des densités des deux matériaux. La continuité de la distribution de la dose au niveau de la première interface 51 est assurée par un ajustement des coefficients $\alpha, \beta, \gamma$. L'ajustement se fait par exemple en un point de la première interface 51 pour lequel la projection équivalente $r$ est connue, à partir de la projection du modèle pilier de la maille 50.

Un deuxième calcul peut utiliser un quatrième fantôme homogène traversé par le faisceau 43, de même matériau que le matériau de la quatrième maille 52.. La distribution de dose dans le quatrième fantôme homogène est utilisée comme quatrième fonction modèle du modèle de diffusion de la quatrième maille 52. La dose déposée dans la quatrième maille 52 provient de particules dont les trajectoires ont été initiées dans la troisième maille 50 par des interactions avec des photons du faisceau 43 se diffusant de manière latérale par rapport au faisceau 43. La dose déposée dans la quatrième maille 52 peut donc être obtenue en utilisant une distribution de doses déposées par le faisceau 43 dans le quatrième fantôme homogène. La fonction de projection du modèle est initialisée comme pour un modèle pilier, pour les coefficients $a, b, c, d, e, f, g, h, i$ de $P$. Une deuxième mise à l'échelle est alors effectuée. Elle s'applique à la troisième dimension de $r$ et donc aux coefficients $g, h, i$ de la matrice de projection $P$. La deuxième mise à l'échelle peut être réalisée par exemple selon un rapport des densités des deux matériaux. La mise à l'échelle permet de prendre en compte les écarts entre les coefficients d'atténuation linéique, correspondant à la décroissance de la dose déposée en fonction de la profondeur $z$. Les coefficients $\alpha, \beta, \gamma$ sont ensuite ajustés en considérant un point de la première interface 51 pour lequel on calcule sa projection $r$. Cette projection peut être déduite de la projection de ce point dans le modèle pilier de la troisième maille 50 selon le principe déjà vu de profondeur équivalente, directement lié à la profondeur radiologique, et de son extension à la notion de distance équivalente au bord du faisceau. Les coefficients $\alpha, \beta, \gamma$ peuvent donc être réglés pour que la distance au bord du faisceau dans le quatrième fantôme homogène soit égale au produit de la distance au bord du faisceau dans le modèle précédent, donc ici de la troisième maille 50, par le ratio des densités électroniques. Un coefficient de pondération sur le modèle de diffusion intervient afin d'assurer une continuité de la distribution de la dose à l'interface entre la troisième et la quatrième maille 50, 52. Le coefficient de pondération peut être déterminé par exemple à partir de la valeur de la dose en un point de la première interface 51.

Un troisième calcul peut utiliser indifféremment la distribution de dose dans le troisième fantôme homogène ou le quatrième fantôme homogène traversé par le faisceau 43. La fonction de projection du modèle est entièrement calculée en résolvant un système pour plusieurs points de la première interface 51 pour lesquels, selon les principes déjà décrit dans les deux calculs précédents en fonction du matériau pris comme référence, les projections $r$ ont été déterminées Sous condition de prendre au moins quatre points différents, la matrice $P$ peut alors être entièrement déterminée. Dans le cas où c'est la distribution de dose dans le quatrième fantôme homogène, c'est-à-dire dans un milieu homogène au matériau de la maille courante, un coefficient de pondération sur le modèle de diffusion intervient afin d'assurer une continuité de la distribution de la dose à la première interface 51 entre la troisième et la quatrième maille 50, 52. Le coefficient de pondération peut être déterminé par exemple à partir de la valeur de dose en un point de l'interface.

La figure 6 représente le faisceau 43 se propageant sur une deuxième interface 60 entre une cinquième maille 61 et une sixième maille 62. La cinquième maille 61 peut comporter un matériau composé d'eau et la sixième maille peut comporter un matériau composé d'os. Le faisceau 43 se propage de manière sensiblement parallèle à la deuxième interface 60 entre deux matériaux de différente composition. Ainsi une partie du faisceau 43 se trouve dans la cinquième maille 61 et une autre partie du faisceau 43 se trouve dans la sixième maille 62. Pour traiter ce cas particulier, il est possible de considérer le faisceau 43 comme un faisceau composé de deux sous-faisceaux 63, 64. Un premier sous-faisceau 63 se propage dans la cinquième maille 61 et un deuxième sous-faisceau 64 se propage dans la sixième maille 62. Par exemple, le faisceau complet 43 peut avoir une section de $1 \times 1$ cm$^2$. Le faisceau complet peut ainsi être découpé en deux sous-faisceaux 63, 64 adjacents de section $0,5 \times 1$ cm$^2$ par exemple.

Pour obtenir une distribution de la dose déposée par le faisceau en un point d'une maille, on somme les contributions des sous-faisceaux composant le faisceau. Les contributions des sous-faisceaux, prennent la forme de modèles piliers ou de modèles de diffusion pour des faisceaux complets. Lesdites contributions comportent une pondération des modèles, de manière à prendre en compte le fait que les sous-faisceaux ne sont que des parties du faisceau complet. Les pondérations se basent sur une modélisation de l'étalement latéral de la dose déposée par chaque sous-faisceau. Ledit étalement latéral est centré sur le sous-faisceau associé.

Par exemple, si l'on considère un plan sensiblement orthogonal à l'axe du faisceau 43, la dose sur le plan peut être la somme de différents modèles de dépôt de doses $H_f(p'_f)$ en milieu homogène, pris à des positions $p_f$ équivalentes aux positions $p$ dans la maille du fantôme hétérogène, lesdits modèle de doses $H_f(p'_f)$ étant pondérés par des poids $\omega_f$ associés à chaque sous-faisceau $f$ :

$$D(p) = \sum_F D_f(p) = \sum_F H_f(p'_f).\omega_f(p) \qquad (1006)$$

où $F$ représente l'ensemble des sous-faisceaux $f$ constituant le faisceau à la profondeur considérée. La somme des pondérations $\omega_f$ de chaque sous-faisceau $f$ est posée égale à un.

Les pondérations $\omega_f$ peuvent par exemple être calculées à partir de fonctions $k_f(p)$ représentant l'étalement de l'énergie dissipée par le rayonnement ionisant provenant d'un sous-faisceau $f$, en fonction de l'écart entre la position $p$ et l'axe du sous-faisceau $f$, selon la formule suivante :

$$\omega_f(p) = \frac{k_f(p)}{\sum_F k_f(p)} \qquad (1007)$$

La formule (1007) est une normalisation des poids $k_f(p)$. Il est à noter que les poids sont des fonctions de l'espace et donc la pondération peut varier selon la position $p$. La formule (1006) peut donc également être interprétée comme la somme simple des modèles de doses $H_f$ modulés respectivement par les fonctions $\omega_f$.

On obtient donc la relation suivante, $f'$ étant un sous-faisceau appartenant à l'ensemble des sous-faisceaux $F$ :

$$D(p) = \sum_{f \in F} \frac{k_f(p)}{\sum_{f' \in F} k_{f'}(p)}.H_f(p'_f) \qquad (1008)$$

et donc

$$D(p) = \frac{1}{\sum_F k_f(p)} \sum_F k_f(p).H_f(p'_f) \qquad (1009)$$

Un premier exemple de fonction $k_f(p)$ peut être une fonction gaussienne valant un au centre du faisceau. En considérant un sous-faisceau f comme rectangulaire, par approximation si besoin, une matrice de covariance $\sum_f$ de la gaussienne peut être définie pour que ces axes principaux soient ceux du rectangle et l'écart-type selon ces axes soit la demi-longueur du côté associé à l'axe. Le centre de la gaussienne peut correspondre au centre du sous-faisceau f. Ensuite, la gaussienne peut être multipliée par la surface du sous-faisceau f à la profondeur considérée. La multiplication par la surface du sous-faisceau f à la profondeur considérée, c'est-à-dire sa section, permet avantageusement de représenter le fait que la contribution du sous-faisceau f est d'autant plus importante que sa section est grande.

On obtient alors la formule suivante pour la fonction $k_f(p)$ :

$$k_f(p) = a_f . e^{-\frac{1}{2}(p-c_f)^T \Sigma_f^{-1}(p-c_f)} \qquad (1010)$$

où $a_f$ est l'aire de la section du sous-faisceau f, $\sum_f$ sa fonction de covariance et $c_f$ son centre.

L'utilisation d'une gaussienne comme fonction de pondération est simple et évite avantageusement des surcharges en temps de calcul.

Une autre fonction $k_f(p)$ peut être utilisée : c'est la fonction de Bell, dite courbe en cloche. En trois dimensions, la fonction de Bell peut prendre la forme suivante :

$$B(p) = \frac{1}{1+\left(\left(p-c_f\right)^T\left(\Sigma_f\right)^{-1}\left(p-c_f\right)\right)^b} \qquad (1011)$$

où b permet de régler la pente de la fonction. Par rapport aux pentes observées dans le matériau, on peut poser b comme étant le produit de cette pente par le déterminant de $\sum_f$ ce qui donne ainsi en moyenne la même pente. Il est également possible de multiplier la hauteur du profil de dose souhaitée par l'aire $a_f$ de la section du faisceau f. On obtient alors une fonction qui modélise bien l'écartement latéral de la distribution de dose et notamment, son centre, son amplitude, c'est à dire le niveau de la dose au centre de la courbe, la largeur de son plateau, et sa pente extérieure.

Toutes les fonctions $k_f(p)$ sont prises en compte dans le calcul de la pondération en un point d'une maille. Et ceci même pour un sous-faisceau f dont le modèle de diffusion n'a pas été calculé car il a été considéré comme négligeable dans la maille courante.

On notera que dans la pratique seul le plan orthogonal à l'axe de propagation du faisceau nous concerne. Cela se conceptualise dans $\sum_f$ par un écart-type infini selon ce troisième axe, et donc une valeur propre nulle dans son inverse, qui est la seule quantité à représenter. Dans la pratique, on pourra avantageusement se contenter de projeter la grandeur $(p-c_f)$ sur le plan d'intérêt et de considérer une matrice de covariance en deux dimensions.

Une autre approche est par exemple de considérer le faisceau 43 comme complet dans chacune des cinquième maille 61 et sixième maille 62. Ensuite une correction peut être appliquée à l'interface.

Une autre possibilité est de disposer de modèles pré calculés pour toutes les dimensions de sous-faisceaux et de les additionner directement.

La figure 7 représente un faisceau 43 traversant deux mailles 71, 72 de manière sensiblement orthogonal à une troisième interface 70 entre les deux mailles 71, 72. Une septième maille 71 peut comporter un milieu composé par exemple d'eau alors qu'une huitième maille 72 peut comporter un milieu différent du milieu de la septième maille 71 composée par exemple d'os. Le faisceau 43 traverse donc deux milieux différents, et donc deux matériaux différents. Plusieurs méthodes peuvent être utilisées afin de calculer, pour le cas représenté sur la figure 7, des éléments correctifs 302, représentés sur la figure 3b, pour les modèles piliers des septième et huitième mailles 71, 72. La correction n'est pas obligatoire mais elle permet d'obtenir des résultats plus fiables.

Une première méthode possible est une méthode utilisant un « shutdown », signifiant littéralement arrêt. Un « shutdown » est un modèle représentant la dose déposée après une interface par des particules mises en mouvement avant celle-ci.

Dans cette méthode, en première approximation, la rétrodiffusion est négligée. Cette approximation est particulièrement efficace lorsque les deux matériaux ont des caractéristiques physiques proches.

De manière générale pour calculer la dose déposée dans la huitième maille 72, après la troisième interface 70, on commence par effectuer le calcul du modèle pilier. Ensuite, on ajoute un élément correctif pour prendre en compte un différentiel de flux électronique à la troisième interface 70. Ainsi l'expression de la dose déposée dans la huitième maille 72 juste après la troisième interface 70 dépend de la nature du matériau de la septième maille 71 et de la nature du matériau de la huitième maille 72. Dans cette méthode, l'élément correctif correspond à un shut down dont l'amplitude est définie à la troisième interface 70 comme valant la différence entre la dose déposée à la troisième interface 70 selon le modèle pilier de la huitième maille 72 et la dose déposée à la troisième interface 70 selon le modèle pilier de la septième maille 71. La continuité de la dose au passage de la troisième interface 70 est ainsi assurée.

Un premier calcul de l'élément correctif se fait à partir d'un premier shutdown dans un matériau homogène correspondant au matériau de la septième maille 71. Pour intégrer que le shutdown est celui d'un autre matériau que celui de la huitième maille 72, une déformation de ce shutdown est appliquée. Pour faire cette déformation, on considère un point de fuite référence qui se trouve en amont de l'interface, par exemple à 5 cm avant, et au centre du faisceau. Pour un point considéré de la huitième maille 72, on calcule sa distance à la troisième interface 70 selon la droite qui passe par ledit point et le point de fuite. On calcule alors la distance équivalente dans le shutdown considéré. Comme dans le cas de la diffusion, la distance équivalente est obtenue en multipliant par le rapport des densités électroniques des deux matériaux. On reporte dans la fonction shutdown, selon une droite identique cette distance à partir de l'interface référence : la troisième interface 71. On obtient alors la valeur du shutdown déformé. On constate que les paramètres de déformation sont différents selon la direction considérée de diffusion des particules et sont tels que la mise à l'échelle est maximale dans la direction de propagation du faisceau 43 et nulle tout le long de l'interface. Dans la pratique, comme dans le cas des modèles de diffusions, cette déformation peut correspondre à un scaling et peut être effectuée par une fonction de projection.

Un deuxième calcul utilise un deuxième shutdown dans un matériau homogène semblable à celui de la huitième maille 72 afin de calculer la dose déposée après la troisième interface 70. Cette méthode considère de manière indépendante chaque position radiale dans la huitième maille 72. La répartition en profondeur du deuxième shutdown homogène est semblable à la répartition d'un troisième shutdown sur les milieux hétérogènes de la septième et huitième maille 71, 72. Cependant, le deuxième shutdown diffère du troisième shutdown en largeur au niveau de la troisième interface 70, car le troisième shutdown est initié par le matériau de la septième maille 71 avant l'interface. De ce fait, le flux responsable du dépôt de dose ne s'étale pas de la même façon au niveau de l'interface. Une première solution pour adapter le deuxième shutdown est de se baser sur une hypothèse de continuité à l'interface en adaptant le deuxième shutdown de façon à le mettre au bon niveau de flux au moins au niveau de l'interface. L'erreur commise est d'autant plus faible que la dose diminue en s'éloignant de l'interface. La première solution prend en considération indépendamment les valeurs de dose déposée en chaque position radiale ($x$, $y$) du troisième shutdown et à rendre continues les valeurs de doses ainsi obtenues en les multipliant par un coefficient de façon à ce qu'elles soient égales aux valeurs du premier shutdown au niveau de la troisième interface 70. On peut donc multiplier le deuxième shutdown à une position ($x$, $y$, $z$) par le rapport suivant :

$$\frac{SD_{11}(x, y, z = 0)}{SD_{22}(x, y, z = 0)} \qquad (1012)$$

avec $SD_{11}$ ($x$, $y$, $z = 0$) représentant la valeur du premier shutdown à la position ($x$, $y$, 0) et $SD_{22}$ ($x$, $y$, $z = 0$) représentant la valeur du deuxième shutdown à la position ($x$, $y$ ,0), le $z$=0 signifiant ici que l'on se trouve à l'interface. Cette méthode peut être utilisée selon deux approches qui ont déjà été décrites précédemment. La première approche se base sur une distribution de dose en milieu homogène en considérant l'hypothèse de l'équilibre électronique sur l'ensemble des mailles 71, 72. Dans cette première approche une compensation est ajoutée à la distribution afin de rendre compte des flux électroniques réels dans la huitième maille 72. Une deuxième approche se base uniquement sur une distribution de dose en milieu homogène correspondant au matériau de la huitième maille 72 à laquelle est ajoutée la dose provenant de la contribution des particules venant de la septième maille 71.

D'autres méthodes de calcul peuvent être employées comme une utilisation du deuxième shutdown en ajustant toutes ses colonnes par continuité. Une colonne d'un shutdown représente les valeurs du shutdown correspondants à une position radiale ($x$, $y$). Il est également possible de choisir de n'utiliser qu'une des colonnes du deuxième shutdown, par exemple une colonne centrale, afin de gagner du temps de calcul et de l'espace mémoire. Pour assurer la continuité à l'interface, il est possible de prendre un rapport de continuité unique sur l'axe du faisceau 43 et d'appliquer ce même rapport à l'ensemble du deuxième shutdown.

Une autre solution est d'utiliser le deuxième shutdown de manière complète et d'ajuster chacune de ses colonnes pour obtenir une continuité à l'interface.

Une autre approche possible est un calcul par pondération des modèles piliers de chaque maille. L'idée principale est de pouvoir passer du modèle pilier de la septième maille 71 se trouvant avant la troisième interface 70 au modèle pilier de la huitième maille 72 se trouvant après la troisième interface 70 par une transition progressive basée sur des pondérations de chacun des modèles piliers. Pour définir les pondérations, on peut utiliser la modélisation suivante du phénomène physique se produisant aux interfaces : Dans une maille donnée, si l'on se trouve suffisamment loin d'une interface, dans une région d'équilibre électronique, un modèle homogène correspondant au matériau de la maille peut être appliqué avec un poids égal à un. Ainsi ce modèle homogène est le seul modèle contribuant à générer la dose aux points d'équilibre électronique. En se rapprochant d'une interface entre deux matériaux, par exemple orthogonale au faisceau, les régimes électroniques deviennent perturbés. L'influence du modèle pilier de la maille située de l'autre côté de l'interface devient alors plus importante au fur et à mesure que l'on se rapproche de l'interface. La pondération appliquée au modèle de la maille courante diminue donc à l'approche de l'interface alors que la pondération appliquée au modèle pilier de la maille située de l'autre côté de l'interface augmente. Les pondérations appliquées aux modèles piliers des mailles situées de part et d'autre de l'interface sont donc complémentaires et leur somme est égale à un en tout point. Les pondérations utilisées sont des fonctions continues selon la première position p, permettant ainsi une évolution continue des modèles de dépôt de doses.

Une pondération utilisée peut être une fonction sigmoïde dont la valeur neutre peut être placée par exemple à deux ou trois millimètres après l'interface. La largeur nécessaire à la transition est de l'ordre de la longueur nécessaire à l'atteinte d'un équilibre électronique depuis l'interface, c'est-à-dire la profondeur où la dose déposée est maximale dans le cas d'un fantôme homogène composé du même matériau que la maille courante. Les pondérations de type sigmoïdes permettent avantageusement d'assurer une transition lente et continue, représentant bien le fait que les modèles ont un impact profond dans les mailles adjacentes, lié au dépôt de dose secondaire, c'est-à-dire par des particules secondaires. Toutefois, la dose secondaire étant très faible, les effets liés à l'instabilité électronique aux abords de l'interface sont très faibles pour la dose secondaire.

D'autres fonctions de transition ayant une influence plus localisée autour de l'interface peuvent être utilisées :

par exemple une fonction linéaire de pondération ou encore une fonction comme $\dfrac{1}{1+(a.z)^3}$ · où $a$ permet de régler la pente et est donc directement lié à la profondeur d'établissement de l'équilibre électronique.

Le choix entre une fonction linéaire et une fonction de type sigmoïde dépend notamment des critères de performances souhaitées pour le procédé selon l'invention.

La méthode utilisant des pondérations est avantageusement très précise dans l'approximation des dépôts de dose à proximité des interfaces entre des matériaux de nature différente.

La figure 8 représente ce qui se passe pour la diffusion des modèles lorsque le faisceau n'est pas parallèle aux interfaces. Comme nous l'avons dit, un modèle de diffusion, pour des raisons géométriques, n'est pas nécessairement valide sur toute la maille courante. Il doit être associé à un domaine de validité. La figure 8 montre l'intérêt d'un tel domaine de validation et un exemple de détermination de ces domaines. Sur la figure 8, le faisceau 43 traverse les mailles 80, 81 et 82. Le faisceau 43 diffuse dans la maille 83. Toutefois cette diffusion est double. Elle se fait à la fois depuis la maille 81 via l'interface 84 et depuis la maille 82 via l'interface 85. Si aucun domaine de validité n'était associé à chaque modèle de diffusion, on les additionnerait et en fait la diffusion du faisceau 43 serait comptée deux fois. Un premier calcul du domaine de validité peut se faire sur des considérations géométriques à partir des plans sensiblement orthogonaux à l'axe de propagation du faisceau et les interfaces par lesquelles on diffuse. Ainsi, le modèle de diffusion du modèle pilier de la maille 81 via l'interface 84 va n'être valide que dans la zone 85. Le modèle de diffusion du modèle pilier de la maille 82 via l'interface 85 va n'être valide que dans la zone 86. Les deux zones de validité sont complémentaires et la diffusion du faisceau 43 dans la maille 83 est prise en compte partout une seule fois.

Une fois ces domaines de validité définis de façon binaire, on peut lisser ces domaines pour rendre plus continues les transitions d'un domaine à l'autre et éviter ainsi des effets de seuils. On peut les lisser linéairement ou selon une forme sigmoïdale par exemple. L'essentiel est que la somme des validités de tous les modèles de diffusion issus d'un même faisceau ou sous faisceau soient toujours inférieure ou égale à un.

Si c'est le choix de la méthode de pondération entre modèles qui est utilisée pour les éléments correctifs, la définition du domaine de validité peut alors être avantageusement intégrée dans la formule de détermination des pondérations.

Une traversée de manière oblique par un faisceau, d'une interface entre deux matériaux différents, produit un déséquilibre en fluence sur le faisceau. Le déséquilibre en fluence est alors répercuté sur la distribution de dose déposée par le faisceau. Les déséquilibres induits sur la dose par une interface oblique sont moins importants que ceux induits sur la fluence primaire, grâce notamment à un effet de compensation entre les différentes positions via les diffusions électroniques. Cependant, des interfaces très inclinées par rapport au faisceau, de l'ordre de soixante dix degrés ou plus, donnent des écarts en dose conséquents, qu'il convient de modéliser.

Une première solution peut être d'enrichir la projection pour adapter le modèle de base à ces situations extrêmes.

Une seconde solution, simple à mettre en oeuvre, est de découper le faisceau en au moins deux sous-faisceaux primaires, ce qui revient à poser deux modèles piliers sur la maille qui ont des profondeurs équivalentes différentes pour un même point de calcul. Cette approche est similaire à une quantification ou un échantillonnage, permettant de ramener la complexité liée à l'interface oblique à deux cas simples à traiter.

Avantageusement, cette approche est simple et n'introduit pas de projections complexes des coordonnées équivalentes sur les modèles homogènes. De plus cette approche nécessite peu de données d'entrées : principalement des modèles de doses dans le cas où le faisceau est sensiblement orthogonal à l'interface. Cette approche est également facilement paramétrable, permettant ainsi de maitriser la complexité du procédé en fonction de la précision souhaitée pour les résultats.

Avantageusement, le procédé de calcul de dose selon l'invention est très rapide. En effet, dans un premier temps, seules des formules de changement de repère ou de projection sont calculées pour chaque maille du fantôme du patient. On dispose alors pour chaque maille d'une distribution de la dose déposée dans cette maille. En regroupant les voxels d'un même matériau homogène en une ou plusieurs mailles, le nombre de données à traiter est ainsi avantageusement réduit et permet des calculs plus rapides.

En effectuant le calcul de dose pour l'ensemble des voxels, le procédé selon l'invention demeure extrêmement rapide.

Avantageusement le procédé selon l'invention peut s'appliquer à des méthodes de hadronthérapie, protonthérapie et radiothérapie électrons.

**Revendications**

1. Procédé de calcul de doses (1) déposées par au moins un faisceau (43) de particules ionisantes sur des voxels d'un fantôme d'un patient, ledit fantôme étant maillé, chaque maille du fantôme comportant des voxels d'un même matériau, ledit procédé de calcul (1) étant **caractérisé en ce qu'**il comporte au moins les étapes suivantes pour chaque faisceau :

   • une première étape (2) de calcul d'au moins une fonction analytique de répartition de doses déposées par le premier faisceau (43) pour chaque maille d'un ensemble de mailles du fantôme, ladite première étape comportant :

      o un premier calcul de fonctions analytiques (301) pour des premières mailles du fantôme traversées par le premier faisceau (300), les fonctions analytiques ainsi obtenues étant des modèles piliers ;
      o un deuxième calcul de fonctions analytiques pour des deuxièmes mailles du fantôme, non traversées par le premier faisceau (310), par diffusion des modèles piliers (311), en parcourant de proche en proche les deuxièmes mailles du fantôme (34), en partant des mailles traversées par le premier faisceau, pour obtenir des modèles de diffusion pour les mailles de l'ensemble de mailles qui ne sont pas traversées par le premier faisceau (310) ;

   • une deuxième étape de calcul de doses (3) sur plusieurs voxels du maillage, la valeur de la dose pour un voxel étant la valeur de la fonction analytique de répartition de doses de la maille à laquelle appartient le voxel, à la position du voxel dans la maille,

   et **en ce que** une fonction analytique est composée d'au moins deux fonctions:

      • une première fonction de projection associant une première position p d'une maille (44, 45) à une deuxième position p' dans un fantôme d'un matériau homogène (41, 42), ledit matériau homogène ayant des caractéristiques semblables aux caractéristiques du matériau des voxels de la maille (44, 45) ; et
      • une deuxième fonction modèle associant à la deuxième position p' dans le fantôme du matériau homogène (41, 42) une dose y étant déposée par un deuxième faisceau (43) semblable au premier faisceau (43), ladite dose étant donnée par un modèle de base pré-calculé.

2. Procédé selon la revendication 1, **caractérisé en ce que** l'ensemble des mailles du fantôme comporte des mailles pour chacune desquelles au moins une des valeurs de la fonction analytique sur la maille, est supérieure à un seuil donné (34).

3. Procédé selon l'une des revendications 1 et 2, **caractérisé en ce que** le modèle de base pré-calculé utilise une distribution de dose obtenue par une simulation selon une méthode de Monte-Carlo.

4. Procédé selon l'une quelconque des revendications 1 à 3, **caractérisé en ce qu'**un modèle de diffusion est composé de trois fonctions :

      - la première fonction de projection ;
      - la deuxième fonction modèle ;

- une troisième fonction de validité associant à une troisième position dans une des deuxièmes mailles, un degré de pondération appliqué à la deuxième fonction modèle.

5. Procédé selon l'une quelconque des revendications 1 à 4, **caractérisé en ce qu'**un calcul de fonctions analytiques de répartition de doses est effectué pour deux mailles adjacentes de différents matériaux, la deuxième interface entre les deux mailles étant traversée de manière oblique par le premier faisceau (43), en utilisant une décomposition du premier faisceau (43) en plusieurs sous-faisceaux ; ledit calcul de fonctions analytiques étant effectué pour chaque sous-faisceau de la même façon que pour un faisceau.

6. Procédé selon l'une quelconque des revendications 1 à 5, **caractérisé en ce qu'**un calcul de fonctions analytiques de répartition de doses étant effectué pour deux mailles adjacentes (61, 62) de différents matériaux, le premier faisceau (43) se propageant de manière sensiblement parallèle à la première interface (61), ledit calcul de fonctions analytiques comporte un calcul d'une fonction analytique par sous-faisceau (63, 64), ledit premier faisceau (43) étant décomposé en plusieurs sous-faisceaux (63, 64), ledit calcul de fonctions analytiques étant effectué pour chaque sous-faisceau de la même façon que pour un faisceau.

7. Procédé selon l'une quelconque des revendications 5 et 6, **caractérisé en ce qu'**une fonction analytique de répartition de doses déposées par le premier faisceau (43) est obtenue par une somme pondérée des fonctions analytiques associées à chaque sous-faisceau (63, 64) du premier faisceau (43), ladite pondération dépendant d'une première position p d'une maille (44, 45).

8. Procédé selon la revendication 7, **caractérisé en ce que** la pondération est déduite d'une normalisation de premiers coefficients issus d'une fonction de forme Gaussienne.

9. Procédé selon la revendication 7, **caractérisé en ce que** la pondération est déduite d'une normalisation de deuxièmes coefficients issus d'une fonction de la forme d'une fonction de Bell.

10. Procédé selon l'une quelconque des revendications 1 à 9, **caractérisé en ce que** des éléments correctifs sont appliqués à une fonction analytique de répartition de doses pour une cinquième maille (71), de matériau différent par rapport à une sixième maille adjacente (72) à la cinquième maille (71), lesdits éléments correctifs modélisant une discontinuité électronique à proximité d'une troisième interface (70) entre la cinquième maille (71) et la sixième maille (72) adjacente.

11. Procédé selon la revendication 10, **caractérisé en ce que** les éléments correctifs sont basés sur des modèles " shutdown ", signifiant littéralement modèles d'arrêt.

12. Procédé selon la revendication 10, **caractérisé en ce que** les éléments correctifs pour une maille sont basés sur une somme pondérée des fonctions analytiques de la maille et des fonctions analytiques des mailles adjacentes à la maille, ladite pondération dépendant d'une première position p dans la maille.

**Patentansprüche**

1. Verfahren zum Berechnen von Dosen (1), die von wenigstens einem Strahl (43) von ionisierenden Partikeln auf Voxeln des Phantoms eines Patienten abgesetzt werden, wobei das Phantom vermascht ist, wobei jede Masche des Phantoms Voxel desselben Materials umfasst, wobei das Berechnungsverfahren (1) **dadurch gekennzeichnet ist, dass** es wenigstens die folgenden Schritte für jeden Strahl beinhaltet:

• einen ersten Schritt (2) des Berechnens von wenigstens einer analytischen Funktion des Austeilens von Dosen, die von dem ersten Strahl (43) für jede Masche eines Maschensatzes des Phantoms abgesetzt wurden, wobei der erste Schritt Folgendes beinhaltet:

○ eine erste Berechnung von analytischen Funktionen (301) für erste Maschen des Phantoms, die von dem ersten Strahl (300) durchstrahlt werden, wobei die so erhaltenen analytischen Funktionen Säulenmodelle sind;
○ eine zweite Berechnung von analytischen Funktionen für zweite Maschen des Phantoms, die nicht von dem ersten Strahl (310) durchstrahlt werden, durch Verbreitung der Säulenmodelle (311), unter schrittweisem Passieren der zweiten Maschen des Phantoms (34), beginnend mit den vom ersten Strahl durchstrahlten Maschen, um Verbreitungsmodelle für die Maschen des Maschensatzes zu erhalten, die nicht von dem ersten Strahl (310) durchstrahlt werden;

• einen zweiten Schritt des Berechnens von Dosen (3) an mehreren Voxeln des Maschennetzes, wobei der Wert der Dosis für ein Voxel der Wert der analytischen Funktion des Austeilens von Dosen der Masche ist, zu der das Voxel gehört, an der Position des Voxels in der Masche,
und dadurch, dass eine analytische Funktion aus wenigstens zwei Funktionen zusammengesetzt ist:
• einer ersten Projektionsfunktion, die eine erste Position p einer Masche (44, 45) mit einer zweiten Position p' in einem Phantom aus einem homogenen Material (41, 42) assoziiert, wobei das homogene Material Charakteristiken ähnlich den Charakteristiken des Materials der Voxel der Masche (44, 45) hat; und
• einer zweiten Modellfunktion, die mit der zweiten Position p' im Phantom aus dem homogenen Material (41, 42) eine Dosis y assoziiert, die durch einen zweiten Strahl (43) ähnlich dem ersten Strahl (43) abgesetzt wird, wobei die Dosis von einem vorberechneten Basismodell gegeben wird.

2. Verfahren nach Anspruch 1, **dadurch gekennzeichnet, dass** der Maschensatz des Phantoms Maschen umfasst, für jede von denen wenigstens einer der Werte der analytischen Funktion auf der Masche größer ist als eine gegebene Schwelle (34).

3. Verfahren nach einem der Ansprüche 1 und 2, **dadurch gekennzeichnet, dass** das vorberechnete Basismodell eine Dosisverteilung benutzt, die durch eine Simulation gemäß einem Monte-Carlo-Verfahren erhalten wird.

4. Verfahren nach einem der Ansprüche 1 bis 3, **dadurch gekennzeichnet, dass** ein Ausbreitungsmodell aus drei Funktionen zusammengesetzt ist:

   - der ersten Projektionsfunktion;
   - der zweiten Modellfunktion;
   - einer dritten Validitätsfunktion, die einen auf die zweite Modellfunktion angewandten Gewichtungsgrad mit einer dritten Position in einer der zweiten Maschen assoziiert.

5. Verfahren nach einem der Ansprüche 1 bis 4, **dadurch gekennzeichnet, dass** eine Berechnung von analytischen Funktionen des Austeilens von Dosen für zwei benachbarte Maschen aus unterschiedlichen Materialien erfolgt, wobei die zweite Grenzfläche zwischen den zwei Maschen von dem ersten Strahl (43) schräg mittels einer Unterteilung des ersten Strahls (43) in mehrere Teilstrahlen durchstrahlt wird, wobei die Berechnung von analytischen Funktionen für jeden Teilstrahl in gleicher Weise erfolgt wie für einen Strahl.

6. Verfahren nach einem der Ansprüche 1 bis 5, **dadurch gekennzeichnet, dass**, wobei eine Berechnung von analytischen Funktionen, die zum Austeilen von Dosen für zwei benachbarte Maschen (61, 62) aus unterschiedlichen Materialien erfolgt, wobei sich der erste Strahl (43) im Wesentlichen parallel zur ersten Grenzfläche (61) ausbreitet, die Berechnung von analytischen Funktionen eine Berechnung einer analytischen Funktion pro Teilstrahl (63, 64) beinhaltet, wobei der erste Strahl (43) in mehrere Teilstrahlen (63, 64) unterteilt wird, wobei die Berechnung von analytischen Funktionen für jeden Teilstrahl in gleicher Weise erfolgt wie für einen Strahl.

7. Verfahren nach einem der Ansprüche 5 und 6, **dadurch gekennzeichnet, dass** eine analytische Funktion des Austeilens von vom ersten Strahl (43) abgesetzten Dosen anhand einer gewichteten Summe der mit jedem Teilstrahl (63, 64) des ersten Strahls (43) assoziierten analytischen Funktionen erhalten wird, wobei die Gewichtung von einer ersten Position p einer Masche (44, 45) abhängig ist.

8. Verfahren nach Anspruch 7, **dadurch gekennzeichnet, dass** die Gewichtung von einer Normalisierung von ersten Koeffizienten abgeleitet wird, die von einer Funktion der Form einer Gaußschen Funktion kommen.

9. Verfahren nach Anspruch 7, **dadurch gekennzeichnet, dass** die Gewichtung von einer Normalisierung von zweiten Koeffizienten abgeleitet ist, die von einer Funktion der Form einer Bell-Funktion kommen.

10. Verfahren nach einem der Ansprüche 1 bis 9, **dadurch gekennzeichnet, dass** Korrekturelemente auf eine analytische Funktion des Austeilens von Dosen für eine fünfte Masche (71) aus einem anderen Material als dem einer der fünften Masche (71) benachbarten sechsten Masche (72) angewandt werden, wobei die Korrekturelemente eine elektronische Diskontinuität in der Nähe einer dritten Grenzfläche (70) zwischen der fünften Masche (71) und der sechsten benachbarten Masche (72) modellieren.

**11.** Verfahren nach Anspruch 10, **dadurch gekennzeichnet, dass** die Korrekturelemente auf "Shutdown"-Modellen basieren, was wörtlich Abschaltmodelle bedeutet.

**12.** Verfahren nach Anspruch 10, **dadurch gekennzeichnet, dass** die Korrekturelemente für eine Masche auf einer gewichteten Summe der analytischen Funktionen der Masche und analytischen Funktionen der der Masche benachbarten Maschen basieren, wobei die Gewichtung von einer ersten Position p in der Masche abhängig ist.

**Claims**

**1.** A method for calculating doses (1) deposited by at least one beam (43) of ionising particles on voxels of a phantom of a patient, said phantom being meshed, each mesh of said phantom comprising voxels of the same material, said calculating method (1) being **characterised in that** it comprises at least the following steps for each beam:

 • a first step (2) of calculating at least one analytical function for spreading doses deposited by said first beam (43) for each mesh of a set of meshes of said phantom, said first step comprising:

  ○ a first calculation of analytical functions (301) for first meshes of said phantom traversed by the first beam (300), the analytical functions thus obtained being pillar models;
  ○ a second calculation of analytical functions for second meshes of said phantom not traversed by the first beam (310), by scattering the pillar models (311), by gradually passing through the second meshes of said phantom (34), starting from the meshes traversed by the first beam, in order to obtain scatter models for the meshes of the set of meshes that are not traversed by the first beam (310);

 • a second step of calculating doses (3) on a plurality of voxels of the meshwork, the value of the dose for a voxel being the value of the analytical function for spreading doses of the mesh to which the voxel belongs, at the position of the voxel in the mesh,
 and **in that** an analytical function is composed of at least two functions:
 • a first projection function associating a first position p of a mesh (44, 45) with a second position p' in a phantom of a homogenous material (41, 42), said homogenous material having characteristics similar to the characteristics of the material of the voxels of the mesh (44, 45); and
 • a second model function associating a dose y with the second position p' in said phantom of the homogenous material (41, 42), with said dose y being deposited by a second beam (43) similar to the first beam (43), said dose being provided by a precalculated base model.

**2.** The method according to claim 1, **characterised in that** the set of meshes of said phantom comprises meshes, for each of which at least one of the values of the analytical function on the mesh is greater than a given threshold (34).

**3.** The method according to any one of claims 1 and 2, **characterised in that** the precalculated base model uses a dose distribution obtained by a simulation according to a Monte Carlo method.

**4.** The method according to any one of claims 1 to 3, **characterised in that** a scatter model is composed of three functions:

 - the first projection function;
 - the second model function;
 - a third validity function associating a degree of weighting applied to the second model function with a third position in one of the second meshes.

**5.** The method according to any one of claims 1 to 4, **characterised in that** a calculation of analytical functions for spreading doses is carried out for two adjacent meshes of different materials, with the second interface between the two meshes being traversed in an oblique manner by the first beam (43) using a decomposition of the first beam (43) into a plurality of sub-beams, with said calculation of analytical functions being carried out for each sub-beam in the same way as for a beam.

**6.** The method according to any one of claims 1 to 5, **characterised in that**, with a calculation of analytical functions for spreading doses being carried out for two adjacent meshes (61, 62) of different materials, the first beam (43) propagating in a manner substantially parallel to the first interface (61), said calculation of analytical functions comprises a calculation of an analytical function per sub-beam (63, 64), said first beam (43) being decomposed into a plurality of sub-beams (63, 64), with said calculation of analytical functions being carried out for each sub-beam in the same way as for a beam.

7. The method according to any one of claims 5 and 6, **characterised in that** an analytical function for spreading doses deposited by the first beam (43) is obtained by a weighted sum of the analytical functions associated with each sub-beam (63, 64) of the first beam (43), with said weighting depending on a first position p of a mesh (44, 45).

8. The method according to claim 7, **characterised in that** the weighting is deduced from a normalisation of first coefficients coming from a function with a Gaussian profile.

9. The method according to claim 7, **characterised in that** the weighting is deduced from a normalisation of second coefficients coming from a function with the profile of a Bell function.

10. The method according to any one of claims 1 to 9, **characterised in that** corrective elements are applied to an analytical function for spreading doses for a fifth mesh (71), of different material relative to a sixth mesh (72) adjacent to the fifth mesh (71), said corrective elements modelling an electronic discontinuity in the vicinity of a third interface (70) between the fifth mesh (71) and the sixth adjacent mesh (72).

11. The method according to claim 10, **characterised in that** the corrective elements are based on "shutdown" models, which literally means stop models.

12. The method according to claim 10, **characterised in that** the corrective elements for a mesh are based on a weighted sum of the analytical functions of the mesh and analytical functions of the meshes adjacent to the mesh, said weighting depending on a first position p in the mesh.

EP 2 453 985 B1

**FIG.1**

20 — Pour chaque voxel du fantôme faisant partie d'une liste de voxels d'intérêt

21 — Identification de la maille comportant le voxel courant

*Passage à un voxel suivant de la liste*

28 —

22 — Pour chaque modèle de la maille identifiée

23 — Calcul par le modèle courant de la dose correspondant à la position du voxel courant dans la maille

26 —

*Passage à un modèle suivant de la maille*

24 — Ajout de la dose calculée par le modèle courant aux doses calculées pour les modèles précédents

25

*Tant qu'il existe d'autres modèles pour la maille*

*Si pas d'autres modèles pour la maille et qu'il reste des voxels à traiter dans la liste*

27 —

# FIG.2a

200 **Pour chaque maille du fantôme**

Passage à une maille suivante du fantôme
210

201 **Pour chaque voxel de la maille courante**

Passage à un voxel suivant de la maille courante
208

202 **Pour chaque modèle de la maille courante**

206
Passage à un modèle suivant de la maille

203 **Calcul par le modèle courant de la dose correspondant à la position du voxel courant dans la maille courante**

204 **Ajout de la dose calculée par le modèle courant aux doses calculées pour les modèles précédents**

205
Tant qu'il existe d'autres modèles pour la maille courante

207 — Si pas d'autres modèles pour la maille courante et qu'il reste des voxels à traiter

209 — Tant qu'il existe d'autres mailles à traiter

# FIG.2b

6

30 — Pour chaque faisceau

Passage au faisceau suivant

38

31 — Initialisation d'une liste de mailles pour lesquelles le modèle est évalué

32 — Pour chaque maille de la liste

33 — Détermination du modèle analytique de la maille courante pour le faisceau courant

Passage à une maille suivante de la liste

37

34 — Identification des mailles adjacentes à la maille courante dans lesquelles le faisceau courant à une influence significative

35 — Ajout des mailles identifiées à la liste des mailles pour lesquelles le modèle est évalué

Lorsque toutes les mailles de la liste ont été traitées — 36

# FIG.3a

Détermination du modèle analytique de la maille courante pour le faisceau courant

| Le faisceau traverse physiquement la maille courante |
300

| Le faisceau ne rentre pas dans la maille courante |
310

| Calcul du modèle pilier |
301

| Calcul d'un modèle de diffusion |
311

| Calcul d'éléments correctifs |
302

| Calcul d'éléments correctifs |
312

303 — | Obtention de modèles analytiques pour la maille courante |

33

FIG.3b

FIG.4

EP 2 453 985 B1

FIG.5

FIG.6

FIG.7

FIG.8

EP 2 453 985 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **ANDREO P.** Monte Carlo techniques in medical radiation physic. *Phys. Med. Biol.,* 1991, vol. 36, 861-920
- **SALVAT F. ; FERNANDEZ-VAREA J. ; ACOSTA E. ; SEMPAU J.** PENELOPE-2006, A Code System for Monte Carlo Simulation of Electron and Photon Transport. *NEA 6222,* ISBN 92-64-02301-1
- **HUIZENGA, H. ; STORCHI, P. R. M.** Numerical calculation of energy deposition by broad high-energy electron beams. *Phys. Med. Biol.,* 1989, vol. 34, 1371-96
- **JANSSEN, J. J. ; RIEDEMAN, D. ; MORAWSKA-KACZYNSKA, M. ; STORCHI, P. R. M. ; HUIZENGA, H.** Numerical calculation of energy deposition by high-energy electron beams: III. Three-dimensional heterogeneous media. *Phys. Med. Biol.,* 1994, vol. 39, 1351-66
- **GIFFORD K.A. ; HORTON J.L. ; WAREING T.A. ; FAILLA G ; MOURTADA F.** Comparison of a finite-element multigroup discrete-ordinates code with Monte Carlo for radiotherapy calculations. *Physics in Medicine and Biology,* 2006, vol. 51, 2253-2265
- **WONG J. ; PURDY J.** On methods of inhomogeneity corrections for photon transport. *Med. Phys.,* 1990, vol. 17, 807-14
- AAPM de 2004 : Tissue inhomogeneity corrections for megavoltage photon beams. *AAPM Report No 85*
- **CLARKSON, J.** A note on depth doses in fields of irregular shape. *Brit. J. Radiol.,* 1941, vol. 14, 265-8
- **CUNNINGHAM J. R.** Scatter-air ratios. *Phys. Med. Biol.,* 1972, vol. 17, 42-51
- **MACKIE T. R. ; SCRIMGER J. W. ; BATTISTA J. J.** A convolution method of calculating dose for 15-MV x rays. *Med. Phys.,* 1985, vol. 12, 188-96
- **AHNESJÔ A.** Collapsed cone convolution of radiant energy for photon dose calculation in heterogeneous media. *Med. Phys.,* 1989, vol. 16, 577-92
- **TILLIKAINEN L. ; HELMINEN H. ; TORSTI T. ; SILJAMÄKI S. ; ALAKUIJALA J. ; PYYRY J. ; ULMER, W.** A 3D pencil-beam-based superposition algorithm for photon dose calculation in heterogeneous media. *Phys. Med. Biol.,* 2008, vol. 53, 3821-39
- **VASSEUR A. ; MAKOVICKA L. ; MARTIN E. ; SAUGET M. ; CONTASSOT-VIVIER S. ; BAHI J.** Dose calculations using artificial neural networks: A feasibility study for photon beams. *Nucl. Instr. and Meth. in Phys. Res. B,* 2008, vol. 266, 1085-93
- **B. BLANPAIN ; D. MERCIER ; J. BARTHE.** Calcul par réseaux de neurones de la dose déposée en radiothérapie par un faisceau fin dans un volume hétérogène. *ctes de la manifestation des jeunes chercheurs en sciences et technologies de l'information et de la communication MaJeSTIC 2007,* 29 Octobre 2007